(19) Europäisches Patentamt
European Patent Office
Office européen des brevets

(11) Veröffentlichungsnummer: **0 000 176**
**B1**

(12) # EUROPÄISCHE PATENTSCHRIFT

(45) Veröffentlichungstag der Patentschrift: **16.09.81**

(21) Anmeldenummer: **78100209.2**

(22) Anmeldetag: **21.06.78**

(51) Int. Cl.³: **C 07 D 213/63,**
C 07 D 213/85,
C 07 D 213/83,
C 07 D 405/12,
C 07 D 413/12,
A 01 N 43/40

(54) **Pyridyloxy-phenoxy-alkancarbonsäurederivate, Verfahren zu deren Herstellung, und deren Verwendung als Herbizide oder als pflanzenwachstumsregulierende Mittel.**

(30) Priorität: **29.06.77 CH 8023/77**

(43) Veröffentlichungstag der Anmeldung:
**10.01.79 Patentblatt 79/1**

(45) Bekanntmachung des Hinweises auf die Patenterteilung:
**16.09.81 Patentblatt 81/37**

(84) Benannte Vertragsstaaten:
**BE CH DE FR GB NL**

(56) Entgegenhaltungen:
**FR - A - 2 288 089**
**FR - A - 2 329 632**
**FR - A - 2 359 129**
**JA - A - 64 160/75**

(73) Patentinhaber: **CIBA-GEIGY AG**
**Patentabteilung Postfach**
**CH-4002 Basel (CH)**

(72) Erfinder: **Rempfler, Hermann, Dr.**
**Allschwilerweg 67**
**CH-4102 Binningen (CH)**
Erfinder: **Föry, Werner, Dr.**
**Benkenstrasse 65**
**CH-4054 Basel (CH)**

**EP 0 000 176 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Pyridyloxy - phenoxy - alkancarbonsäurederivate, Verfahren zu deren Herstellung, und deren Verwendung als Herbizide oder als pflanzenwachstumsregulierende Mittel

Die Erfindung betrifft Pyridyloxy - phenoxy - alkancarbonsäurederivate, welche eine herbizide und das Pflanzenwachstum regulierende Wirkung zeigen, Verfahren zu ihrer Herstellung, ferner Mittel, die diese Verbindungen als Wirkstoffe enthalten, sowie die Verwendung der Wirkstoffe oder der sie enthaltenden Mittel zur selektiven Bekämpfung von Unkräutern und zur Regulierung des Pflanzenwachstums.

Die erfindungsgemäßen Wirkstoffe entsprechen der Formel I

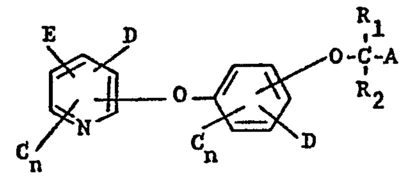

(I)

worin

A die Cyanogruppe, einen Rest —COB oder einen gegebenenfalls durch Methyl ein- oder mehrfach substituierten 2-Oxazolinrest,

B einen Rest $—OR_3$, $—SR_4$ oder $—NR_5R_6$,

C je Halogen, $C_1—C_4$-Alkyl oder Nitro,

D je Wasserstoff, dasselbe wie C, $C_1—C_4$-Alkoxy, $C_1—C_4$-Alkylthio, Cyano, $C_1—C_4$-Alkylsulfinyl, $C_1—C_4$-Alkylsulfonyl, Thiocarbamid ($—CSNH_2$), Mono- oder Di- $C_1—C_4$-alkylsulfamoyl,

E Wasserstoff, dasselbe wie C, Mono- oder Di-$C_1—C_4$-Alkylamin, $C_1—C_5$-Alkoxycarbonyl, $C_2—C_8$-Alkoxyalkyl, Cyano, Trifluoromethyl,

n 0, 1 oder 2

$R_1$ Wasserstoff, $C_1—C_4$-Alkyl, $C_2—C_8$-Alkoxyalkyl, $C_1—C_4$-Haloalkyl,

$R_2$ Wasserstoff, $C_1—C_4$-Alkyl, Carbonyl, $C_1—C_5$-Alkoxycarbonyl,

$R_3$ Wasserstoff oder

das Kation einer Base $\frac{1}{m} M^{m\oplus}$,

wobei

M ein Alkali-, Erdalkali-Kation oder ein Fe-, Cu-, Zn-, 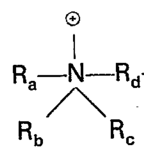-Kation,

m als ganze Zahl 1, 2 oder 3 die Wertigkeit des Kations berücksichtigt, während $R_a$, $R_b$, $R_c$ und $R_d$ unabhängig voneinander Wasserstoff, Benzyl oder einen gegebenenfalls durch —OH, $—NH_2$ oder $C_1—C_4$-Alkoxy substituierten $C_1—C_4$-Alkylrest bedeuten.

Weiter bedeutet

$R_3$ einen $C_1—C_{18}$-Alkyl-Rest, der unsubstituiert oder gegebenenfalls substituiert ist durch Halogen, Nitro, Cyano, $C_1—C_8$-Alkoxy, $C_2—C_8$-Alkoxyalkoxy, $C_3—C_6$-Alkenyloxy, $C_1—C_8$-Alkylthio, $C_2—C_8$-Alkanoyl, $C_2—C_8$-Acyloxy, $C_2—C_8$-Alkoxycarbonyl, Carbamoyl, Bis-($C_1—C_4$-alkyl)-amino, Tris-($C_1—C_4$-alkyl)-ammonio, $C_3—C_8$-Cycloalkyl, $C_3—C_8$-Cycloalkenyl, gegebenenfalls auch durch einen unsubstituierten oder seinerseits durch Halogen, $C_1—C_4$-Alkyl, $C_1—C_4$-Alkoxy, ein- oder mehrfach substituierten Phenyl-, Phenoxy-, Oxiranyl-, Piperidino-, Morpholino-, Pyrrolidino- oder Furylrest,

— einen unsubstituierten oder ein- bis vierfach durch Halogen oder einen durch Phenyl oder Methoxycarbonyl substituierten $C_3—C_{10}$-Alkenylrest,

— einen $C_3—C_8$-Alkinyl-Rest,

— einen gegebenenfalls durch Halogen oder $C_1—C_4$-Alkyl substituierten $C_3—C_{12}$-Cycloalkyl-Rest,

— einen $C_3—C_8$-Cycloalkylen-Rest,

einen Phenylrest, der unsubstituiert oder durch Halogen, $C_1—C_4$-Alkyl, $C_1—C_4$-Alkoxy, $C_1—C_4$-Alkylthio, $—NO_2$, $—CF_3$, —COOH, —CN, —OH, $—SO_3H$, $—NH_2$ oder $—NH(C_1—C_4$-Alkyl) oder $—N(C_1—C_4\text{-Alkyl})_2$ ein- oder mehrfach substituiert ist,

— einen Oxiran-, Piperidino-, Morpholino-, Pyrrolidino- oder Furan-Ring,

$R_4$ einen $C_1—C_{12}$-Alkyl-Rest, der unsubstituiert oder gegebenenfalls substituiert ist durch $C_1—C_8$-Alkoxy, $C_2—C_8$-Alkoxycarbonyl, $C_3—C_8$-Cycloalkyl, gegebenenfalls auch durch einen unsubstituierten oder seinerseits durch Halogen, $C_1—C_4$-Alkyl, $C_1—C_4$-Alkoxy ein- oder mehrfach substituierten Phenyl-, Piperidino-, Morpholino-, Pyrrolidino- oder Furan-Ring,

— einen $C_3—C_{12}$-Alkenyl-Rest;

— einen gegebenenfalls durch $C_1$—$C_4$-Alkyl substituierten $C_3$—$C_{12}$-Cycloalkyl-Rest,
— einen Phenylrest, der unsubstituiert oder durch Halogen, $C_1$—$C_4$-Alkyl, $C_1$—$C_4$-Alkoxy, $C_1$—$C_4$-Alkylthio, —$NO_2$, —$CF_3$ ein- oder mehrfach substituiert ist,
— einen Piperidino-, Morpholino-, Pyrrolidino- oder Furan-Ring,

$R_5$ und $R_6$ unabhängig voneinander je Wasserstoff,
— $C_1$—$C_8$-Alkyl, das durch Halogen, Hydroxyl oder Cyan substituiert und/oder dessen Kette durch Sauerstoff oder Carboxyoxy unterbrochen sein kann,
— $C_3$—$C_6$-Alkenyl, —Alkinyl oder —Cycloalkyl,
— Phenyl oder Benzyl, das durch Halogen, $C_1$—$C_4$-Alkyl, $C_1$—$C_4$-Alkoxy, —$NO_2$ oder —$CF_3$ substituiert sein kann,

$R_5$ und $R_6$ zusammen mit dem Stickstoffatom, an das sie gebunden sind, auch einen Piperidino-, Morpholino- oder Pyrrolidino-Ring;
wobei die Bestimmung gilt, dass der Alkancarbonsäurerest

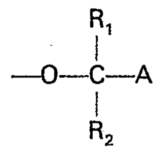

nicht in para-Stellung zu einem gegebenenfalls substituierten 2-Pyridyloxyrest

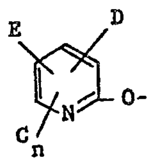

stehen kann.

Die Alkylreste in dieser Formel können verzweigt oder unverzweigt sein und enthalten die angegebene Anzahl Kohlenstoffatome. Die Reste $R_5$ und $R_6$ sind Bevorzugt Wasserstoff und $C_1$—$C_4$-Alkylreste.

Die erfindungsgemäßen Wirkstoffe der Formel I besitzen Herbizidwirkung, vor allem bei postemergenter Anwendung und können in mono- und dikotylen Kulturen als Unkrautmittel eingesetzt werden.

Ferner besitzen sie günstige wachstumsregulierende Effekte (Wuchshemmung). Insbesondere hemmen sie das Wachstum von dicotylen Pflanzen. Beispiele für die nutzbringende Anwendung der erfindungsgemässen Verbindungen sind z.B.

— die Reduktion des vegetativen Wachstums bei Soja und ähnlichen Leguminosen, was zu einer Ertragssteigerung dieser Kulturen führt;
— die Hemmung des unerwünschten Wuchstums von Geiztrieben bei Tabak, dessen Haupttrieb man geschnitten hat, was der Ausbildung grösserer und schönerer Blätter zugute kommt;
— die Hemmung des Wachstums von Gras und dikotyledonen Pflanzen, wie Obstbäume, Zierbäume, Gebüsche und Hecken, zwecks Einsparung an Schnittarbeit.

Die erfindungsgemäßen Verbindungen sind wenig giftig für Warmblüter und deren Applikation wirft keine Probleme auf. Die Aufwandmenge liegt zwischen 0.1 und 5 kg pro Hektar.

Die Herstellung der Verbindungen der Formel I erfolgt nach an sich bekannten Methoden.

Nach einem ersten dieser Verfahren setzt man ein gegebenenfalls substituiertes Halogen-pyridin der Formel II

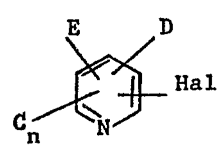 (II)

worin C, D, E und n die unter Formel I gegebene Bedeutung haben und Hal ein Halogenatom, vorzugsweise Chlor oder Brom bedeutet, mit einem Hydroxy - phenoxy - alkancarbonsäurederivat der Formel III

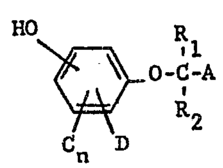 (III)

worin A, C, D, $R_1$ und $R_2$ die unter Formel I gegebene Bedeutung haben, in Gegenwart eines säurebindenden Mittels um.

Solche Umsetzungen sind an sich bekannt und ihre genaue Durchführung kann in Textbüchern wie z.B. in Houben-Weyl, Band 3, Seiten 85 ff nachgelesen werden.

Gemäss einem zweiten Verfahren stellt man die Pyridyloxy - phenoxy - alkancarbonsäurederivate der Formel I her, indem man einen Pyridyloxy-hydroxyphenyläther der Formel IV

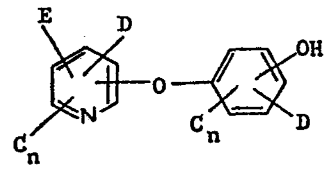

(IV)

worin C, D, E und n die unter Formel I gegebene Bedeutung haben, mit einem $\alpha$-Halogencarbonsäurederivat der Formel V

$$\begin{array}{c} R_1 \\ | \\ Hal—C—A \\ | \\ R_2 \end{array} \qquad (V)$$

worin A, $R_1$ und $R_2$ die unter Formel I gegebene Bedeutung haben und Hal ein Halogenatom, vorzugsweise Chlor oder Brom bedeutet, in Gegenwart eines säurebindenden Mittels umsetzt.

Die genannten Umsetzungen können in An- oder Abwesenheit von gegenüber den Reaktionsteilnehmern inerten Lösungs- oder Verdünnungsmitteln durchgeführt werden. Bevorzugt sind polare organische Lösungsmittel wie Methyläthylketon, Acetonitril, Dimethylformamid, Dimethylsulfoxid etc. Die Reaktionstemperaturen liegen zwischen 0 und 200°C und die Reaktionsdauer beträgt je nach gewählter Umsetzungstemperatur und Lösungsmittel zwischen 1/4 Stunden und mehreren Tagen. Man arbeitet in der Regel bei Normaldruck oder einem leichten Ueberdruck. Als säurebindende Mittel für die Umsetzung kommen anorganische, wie z.B. NaOH, KOH, $NaOCH_3$, NaH, $K_2CO_3$, $NaCO_3$ oder Kalium-tert. butylat aber auch organische Basen in Betracht.

Die Ausgangsstoffe der Formel II bis V sind teilweise bekannt. Noch nicht beschriebene Ausgangsstoffe dieser Formeln lassen sich nach üblichen Verfahren und Techniken leicht herstellen.

Substituierte 2-Halogen-pyridin gemäss Formel II können u.a. leicht aus den entsprechenden 2-Pyridinolen hergestellt werden, welche teilweise bekannt sind. Ausgangsprodukte der Formel III können hergestellt werden, indem man z.B. einen Monobenzyläther von Hydrochinon, Resorcin oder Brenzkatechin mit einem $\alpha$-Halogen-carbonsäurederivat, vorzugsweise einem Ester der Formel V umsetzt und die Benzyl-phenyläther-Bindung durch katalytische Hydrierung spaltet z.B. mit einem Palladium-auf-Kohle-Katalysator, wobei der Benzylrest als Toluol weggeht.

Durch Umsetzung von Hydrochinon, Resorcin oder Brenzkatechin mit Halogenpyridinen in äquimolaren Mengen und in Gegenwart einer Base kann man zu den Ausgangsproduktender Formel IV gelangen.

Carbonsäurederivate der Formel V sind ebenfalls bekannt. Als deren einfachste Vertreter seien z.B. die Chloressigsäure, deren Ester, Thioester, Amide und Hydrazide erwähnt. Es kommen aber auch andere gemäss $R_1$ und $R_2$ substituierte $\alpha$ - Halogencarbonsäure - derivate in Betracht.

Die nachfolgenden Beispiele veranschaulichen das erfindungsgemässe Verfahren für willkürlich ausgewählte Wirkstoffe der Formel I. Weitere in entsprechender Weise hergestellte Wirkstoffe sind in den anschliessenden Tabellen aufgeführt. Temperaturangaben beziehen sich jeweils auf Celsius-Grade.

Diese Pyridyloxy-phenoxy-alkancarbonsäurederivate der Formel I sind stabile Verbindungen, welche in üblichen organischen Lösungsmitteln, wie Alkoholen, Aethern, Ketonen, Dimethylformamid oder Dimethylsulfoxid löslich sind.

## Beispiel 1

$\alpha$[3-(3'-Chlor-5'-cyano-6'-methyl-pyrid-2'-yl)-oxyphenoxy]-propionsäure-methylester

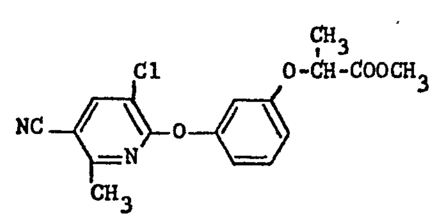

Zu einer Dispersion von 2,6 g (0,11 Mol) Natriumhydroxid in 100 ml Dimethylsulfoxid werden 19,6 g (0,1 Mol) 3 - Hydroxy - phenoxy - $\alpha$ - propionsäuremethylester, gelöst in 50 ml Dimethylsulfoxid, unter Kühlen zugetropft. Wenn sich alles Natriumhydrid gelöst hat, werden 18,7 g (0,1 Mol) 2,3 - Dichlor - 5 - cyano - 6 - methyl - pyridin zugegeben. Nach 8-stündigem Rühren bei Raumtemperatur wird das Dimethylsulfoxid im Vakuum abgedampft, der Rückstand mit 500 ml Eiswasser versetzt und mit Aether ausgeschüttelt. Die Aetherextrakte werden über Magnesiumsulfat getrocknet und dann eingedampft. Der Rückstand wird aus Aether/Petroläther umkristallisiert. Man erhält 20,1 g (58% der Theorie) $\alpha$ - [3 - (3′ - Chlor - 5′ - cyano - 6′ - methyl - pyrid - 2′ - yl) - oxy - phenoxy] - propionsäuremethylester vom Schmelzpunkt 106—109°.

Beispiel 2

$\alpha$-[3-(3′,5′-Dichlorpyrid-2-yl)-oxy-phenoxy]-propionsäure-methylester

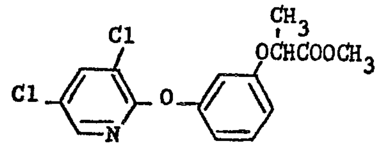

30,8 g (0,1 Mol) 3-(3′,5′-Dichlorpyrid-2′-yl)-oxy-phenol 18,2 g (0,13 Mol) Kaliumcarbonat und 22 g $\alpha$-Brompropionsäuremethylester werden in 300 ml Aethylmethylketon während 3 Stunden unter Rückfluss erhitzt. Dann werden die anorganischen Salze abfiltriert und das Filtrat eingedampft. Durch Umkristallisieren aus Aether/Petroläther erhält man 29 g (70% der Theorie) Titelprodukt mit dem Schmelzpunkt 58—60°.

Beispiel 3

$\alpha$-[3-(3′,5′-Dichlorpyridyl-2′-oxy)-4-chlorphenoxypropionsäure-methylester

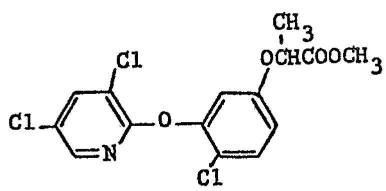

17,3 g (0,05 Mol) $\alpha$-[3-(3′,5′-Dichlorpyrid-2-yl)-oxy-phenoxy]-propionsäure-methylester (siehe Beispiel 2) werden in 260 ml Tetrachlorkohlenstoff und 17,3 ml Eisessig gelöst. Nach Zugabe einer katalytischen Menge Antimontrichlorid werden bei 50° 3,5 g Chlor eingeleitet. Nach einer Stunde wird die Lösung eingedampft und das zurückbleibende Oel aus Aether/Petroläther umkristallisiert. Man erhält so das Titelprodukt vom Schmelzpunkt 76—78°.

Beispiel 4

3-(3′,5′-Dichlorpyridyl-2′-oxy)-6-chlorphenylacetat (Zwischenprodukt)

Eine Mischung von 256 g 3-(3′,5′-Dichlorpyridyl-2′-oxy)-phenol, 1,9 Liter Eisessig und 280 ml Acetanhydrid wird während 12 Stunden bei Rückflusstemperatur gerührt. Die Lösung wird auf 40° gekühlt. Dann wird unter Rühren 81 g Natriumacetat zugegeben und während 7 Stunden bei 40° 106 g Chlorgas eingeleitet. Das überschüssige Chlorgas wird durch ein 2-stündiges Durchleiten von Stickstoff entfernt und die Lösung eingedampft.

Die Essigesterlösung (1000 ml) des öligen Rückstandes wird mit Wasser und gesättigter $NaHCO_3$-Lösung gewaschen, getrocknet und eingedampft. Man erhält 343 g der Titelverbindung als braunes Oel, das mit 300 ml Petroläther verrieben, spontan kristallisiert. Smp.: 71—75°C.

Beispiel 5

3-(3′,5′-Dichlorpyridyl-2′-oxy)-6 resp. -4-Chlorphenol (Zwischenprodukt)

Eine Suspension von 2,26 g pulverisiertem KOH und 10 g 3 - (3′,5′ - Dichlorpyridyl - 2′ - oxy) - 6 - phenylacetat in 30 ml absolutem Methanol wird unter Stickstoffatmosphäre während 20 Minuten bei 60° gerührt, auf Raumtemperatur gekühlt, mit 3,7 ml konzentrierter Salzsäure sauer gestellt und eingedampft.

Die Toluollösung (50 ml) des Rückstandes wird mit Wasser gewaschen, getrocknet, über Aktivkohle filtriert und eingedampft. Man erhält 8,4 g eines Oels, das aus einem Gemisch des -6-Chlor- und -4-Chlorphenolisomeren im Verhältnis 7:3 besteht.

Beispiel 6

$\alpha$-[3-(3′,5′-Dichlorpyridyl-2′-oxy)-6-chlorphenoxy]-propionsäure-methylester

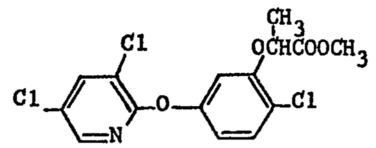

25 g (0.085 Mol) eines Gemisches aus 3 - (3′,5′ - Dichlorpyridyl - 2′ - oxy) - 6 - chlorphenol und 3 - (3′,5′ - Dichlorpyridyl - 2′ - oxy) - 4 - chlorphenol (hergestellt nach Beispiel 5) 14,2 g (0.085 Mol) α - Brompropionsäure-methylester und 13,8 g (0.1 Mol) Kaliumcarbonat werden in 250 ml Methyläthylketon 6 Stunden unter Rückfluss erhitzt. Die anorganischen Salze werden abfiltriert und das Filtrat eingedampft. Das zurückbleibende gelbe Oel wird aus Aether/Petroläther umkristallisiert. Man erhält so 30,1 g (94% der Theorie) isomerenfreies Titelprodukt von Schmelzpunkt 82—83°.

Beispiel 7

3-(3′,5′-Dichlorpyridyl-2′-oxy)-6-brom-phenylacetat (Zwischenprodukt)

Eine Gemisch aus 205 g 3-(3′,5′-Dichlorpyridyl-2′-oxy)-phenol, 1,1 Liter Eisessig und 224 ml Acetanhydrid wird während 12 Stunden bei Rückflusstemperatur gerührt. Die Lösung wird auf 60° gekühlt, dann wird unter Rühren 64,8 g Natriumacetat zugegeben und während $5\frac{3}{4}$ Stunden eine Lösung von 102 ml Brom in 400 ml Eisessig bei 60° zugetropft. Die Lösung wird eingedampft, der Rückstand in Essigester gelöst und die organische Phase nacheinander mit Wasser und gesättigtem $NaHCO_3$-Lösung gewaschen. Die vereinigten Essigesterextrakte werden getrocknet und eingedampft. Man erhält 302,5 g eines Oels, das mit 500 ml Petroläther (Sdp. 60—90°) überschichtet kristallisiert.

Das Titelprodukt schmilzt nach Umkristallisieren aus Cyclohexan bei 106—107°.

Beispiel 8

3-(3′,5′-Dichlorpyridyl-2′-oxy)-6-brom-phenol

Eine Suspension von 28,0 g pulverisiertem KOH und 153,5 g 3 - (3′,5′ - Dichlorpyridyl - 2′ - oxy) - 6 - brom - phenylacetat in einem Liter absolutem Methanol wird unter Stickstoffatmosphäre während 20 Minuten bei 60° gerührt, eingeengt und bei Raumtemperatur mit konzentrierter Salzsäure sauer gestellt und vollständig eingedampft. Die Toluollösung (400 ml) des Rückstandes wird mit Wasser gewaschen, getrocknet und eingedampft. Der ölige Rückstand wird mit Petroläther/Cyclohexan verrieben und filtriert. Man erhält 137 g des kristallinen Titelproduktes. Smp.: 93—95°.

Beispiel 9

α-[3-(3′,5′-Dichlorpyridyl-2′-oxy)-6-bromophenoxy]-proprionsäure-methylester

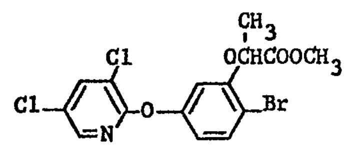

50,2 g (0,15 Mol) 3 - (3′,5′ - Dichlorpyridyl - 2′ - oxy) - 6 - bromphenol (hergestellt gemäss Beispiel 8) 27,5 g (0,165 Mol) α - Brompropionsäure - methylester und 27,6 g (0,2 Mol) Kaliumcarbonat werden in 250 ml Methyläthylketon 5 Std. unter Rückfluss erhitzt. Die anorganischen Salze werden abfiltriert und das Filtrat eingedampft. Das rötliche Oel wird aus Aether/Petroläther umkristallisiert. Man erhält 58 g (91,8% der Theorie) der Titelverbindung mit einem Schmelzpunkt von 75°.

Beispiel 10

α-[3-(3′,5′-Dichlorpyridyl-4′-oxy)-6-chlorphenoxy]-propionsäure-methylester

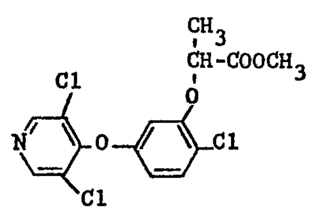

Eine Gemisch aus 4,1 g 3-(3′,5′-Dichlorpyridyl-4′-oxy)-6-chlorphenol, 2,15 g Kaliumcarbonat, 1,74 ml α-Brompropionsäuremethylester und 15 ml Aethylmethylketon wird 4 Stunden bei Rückflusstemperatur gerührt, filtriert und eingedampft.

Man erhält 5 g der Titelverbindung als Oel.

$n_D^{23}$: 1,5587

Beispiel 11

α-[3-(3′,5′-Dichlorpyridyl-2′-oxy)-6-nitrophenoxy]-propionsäure-methylester

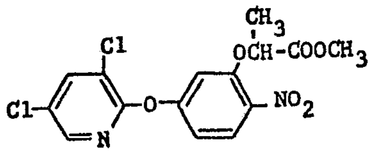

30,1 g (0,1 Mol) 3-(3′,5′-Dichlorpyridyl-2′-oxy)-6-nitrophenol, 16,7 g (0,1 Mol) α-Brompropionsäure-methylester und 20,7 g (0,15 Mol) Kaliumcarbonat werden in 400 ml Methyläthylketon während 14 Stunden unter Rückfluss erhitzt. Die anorganischen Salze werden abfiltriert, das Filtrat eingedampft und das verbleibende braune Oel in Chloroform aufgenommen und über eine kurze Kieselgelsäule filtriert. Man erhält so 22,4 g (58% der Theorie) Titelprodukt, das nach Umkristallisieren aus Hexan bei 102° schmilzt.

TABELLE 1

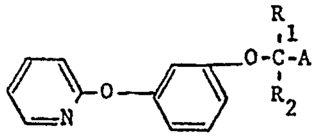

| No. | 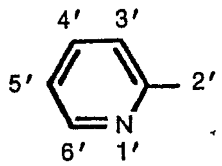 | 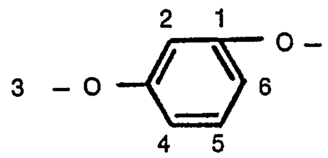 | $R_1$<br>\|<br>$-C-A$<br>\|<br>$R_2$ | Physikalische Konstante |
|---|---|---|---|---|
| 1 | 3′ CN | – | $CH_3$<br>\|<br>$-CHCOOCH_3$ | Smp. 76–78° |
| 2 | 3′CN,5′Cl | 6-Cl | $CH_3$<br>\|<br>$-CHCOOCH_3$ | |
| 3 | 3′CN,5′Cl | – | $CH_3$<br>\|<br>$-CHCOOC_2H_5$ | |
| 4 | 3′Cl,5′Cl | – | $CH_3$<br>\|<br>$-CHCOOC_2H_5$ | |
| 5 | 3′Cl,5′Cl | 6 $NO_2$ | $CH_3$<br>\|<br>$-CHCOOC_2H_5$ | |
| 6 | 3′Cl,5′Cl | 6 Cl | $CH_3$<br>\|<br>$-CHCOOC_2H_5$ | Smp. 53–56° |
| 7 | 3′Cl,5′CN,6′$CH_3$ | 6 $NO_2$ | $CH_3$<br>\|<br>$-CHCOOCH_3$ | Smp. 144–145° |
| 8 | 5′CN,6′$CH_3$ | – | $CH_3$<br>\|<br>$-CHCOOCH_3$ | Smp. 81–83° |
| 9 | 3′Cl,5′Cl,6′Cl | – | $CH_3$<br>\|<br>$-CHCOOCH_3$ | Oel $n_D^{30}$ 1.5723 |
| 10 | 4′$COOCH_3$,6′Cl | – | $CH_3$<br>\|<br>$-CHCOOCH_3$ | Smp. 61–64° |
| 11 | 3′Cl,5′Cl,6′$CH_3$ | – | $CH_3$<br>\|<br>$-CHCOOCH_3$ | Smp. 71–75° |
| 12 | 6′Cl | – | $CH_3$<br>\|<br>$-CHCOOCH_3$ | |

TABELLE 1 (Fortsetzung)

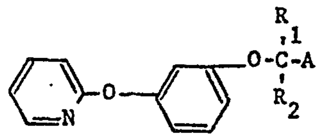

| No. | 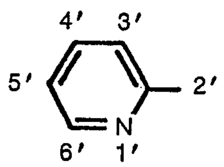 | 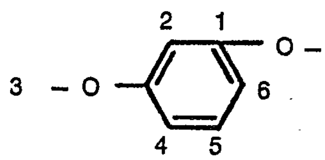 | $R_1$<br>$-C-A$<br>$R_2$ | Physikalische Konstante |
|---|---|---|---|---|
| 13 | 3'CN,4'$CH_3$,6'Cl | – | $CH_3$<br>$-CHCOOCH_3$ | Smp. 61–66° |
| 14 | 3'CN,4'$CH_3$,6'$CH_3$ | – | $CH_3$<br>$-CHCOOCH_3$ | |
| 15 | 5'Br | – | $CH_3$<br>$-CHCOOCH_3$ | |
| 16 | 3'$NO_2$,6'Cl | – | $CH_3$<br>$-CHCOOCH_3$ | Smp. 95–97° |
| 17 | 3'Cl,5'$CSNH_2$,6 $CH_3$ | – | $CH_3$<br>$-CHCOOCH_3$ | |
| 18 | 5'$NO_2$ | – | $CH_3$<br>$-CHCOOCH_3$ | |
| 19 | 5'$NO_2$ | 6 $NO_2$ | $CH_3$<br>$-CHCOOCH_3$ | |
| 20 | 6'Br | – | $CH_3$<br>$-CHCOOCH_3$ | |
| 21 | 3'Cl,5'Cl | 5 $CH_3$ | $CH_3$<br>$-CHCOSCH_2COOCH_3$ | |
| 22 | 3'Cl,5'Cl | 2 $CH_3$,5 $CH_3$ | $CH_3$<br>$-CHCOOCH_3$ | |
| 23 | 3'Cl,5'Cl | – | $-CH_2COOC_2H_5$ | |
| 24 | 3'Cl,5'Cl | – | $CH_3$<br>$-CHCOOCH_2-CH=CH_2$ | |

TABELLE 1 (Fortsetzung)

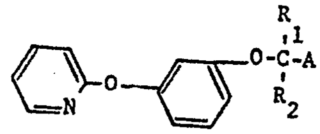

| No. | 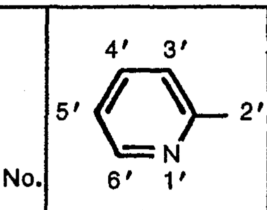 | 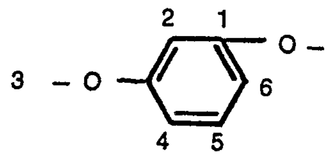 | $R_1$<br>\|<br>$-C-A$<br>\|<br>$R_2$ | Physikalische Konstante |
|---|---|---|---|---|
| 25 | 3'CN,5'Cl | – | $CH_3$<br>\|<br>$-CHCOOCH_2-C\equiv CH$ | |
| 26 | 3'CN,5'Cl | – | $CH_3$<br>\|<br>$-CHCOOC_4H_9$ | |
| 27 | 5'$NO_2$ | 4 Cl,6 Cl | $CH_3$<br>\|<br>$-CHCOOCH_3$ | |
| 28 | 3'Cl,5' Cl | 5 CN | $CH_3$<br>\|<br>$-CHCOOisoC_3H_7$ | |
| 29 | 3'Cl,5'Cl | 4 Cl,6 Cl | $CH_3$<br>\|<br>$-CHCOOCH_3$ | Smp. 83–86° |
| 30 | 3'Cl,5'Cl | 4 Cl | $CH_3$<br>\|<br>$-CHCONHCH_2COOC_2H_5$ | |
| 31 | 3'Cl,5'CN | – | $CH_3$<br>\|<br>$-CHCOOCH_3$ | |
| 32 | 3'Cl,5'CN | 6 Cl | $CH_3$<br>\|<br>$-CHCOOCH_3$ | Smp. 109–111° |
| 33 | 3'Cl,5'Cl | – | $CH_2OCH_3$<br>\|<br>$-CHCOOCH_3$ | |
| 34 | 3'Cl,5'Cl | – | $CH_3$<br>\|<br>$-CHCOOH$ | Smp. 92–93° |

TABELLE 1 (Fortsetzung)

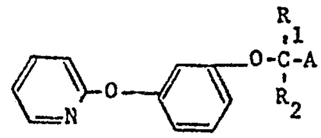

| No. | 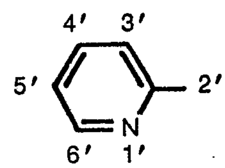 | 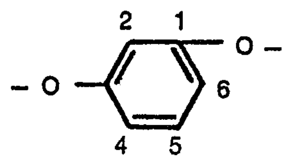 | –CH–A<br>$CH_3$<br>A = | Physikalische Konstante |
|---|---|---|---|---|
| 35 | 3'Cl,5'Cl,6'$CH_3$ | 6 Cl | $-COOCH_3$ | Smp. 94–96° |
| 36 | 3'Cl,5'Cl,6'$CH_3$ | 6 $NO_2$ | $-COOCH_3$ | Smp. 100° |
| 37 | 3'CN,5'Br | 6 Cl | $-COOCH_3$ | Smp. 135–137° |
| 38 | 3'CN,5'Br | 6 $NO_2$ | $-COOCH_3$ | |
| 39 | 5'Cl | 6 Cl | $-COOCH_3$ | Sdp. 145–150°<br>0,04 Torr |
| 40 | 5'Cl | 6 Cl | $-COOC_2H_5$ | |
| 41 | 5'Cl | 6 Cl | $-COOiC_3H_7$ | |
| 42 | 5'Cl | 6 Cl | $-COOiC_4H_9$ | |
| 43 | 5'Cl | 6 Cl | $-COOsec.C_4H_9$ | |
| 44 | 5'Cl | 6 Cl | $-COSCH_3$ | |
| 45 | 5'Cl | 6 Cl | $-COSCH_2CH{=}CH_2$ | |
| 46 | 5'Cl | 6 $NO_2$ | $-COOCH_3$ | |
| 47 | 5'Cl | 6 $NO_2$ | $-COOiC_3H_7$ | |
| 48 | 5'Br | 6 Cl | $-COOCH_3$ | |
| 49 | 5'Br | 6 Cl | $-COOC_2H_5$ | |
| 50 | 5'Br | 6 Cl | $-COOiC_3H_7$ | |
| 51 | 5'Br | 6 Cl | $-COOsec.C_4H_9$ | |
| 52 | 5'Br | 6 Cl | $-COSCH_3$ | |
| 53 | 5'Br | 6 Cl | $-CON(C_2H_5)_2$ | |
| 54 | 5'Br | 6 Cl | $-CONHCH_2.CH{=}CH_2$ | |
| 55 | 5'Br | 6 Cl | $-COSCH_2COOCH_3$ | |
| 56 | 5'Br | 6 $NO_2$ | $-COOCH_3$ | |
| 57 | 5'Br | 6 $NO_2$ | $-COSCH_3$ | |

TABELLE 1 (Fortsetzung)

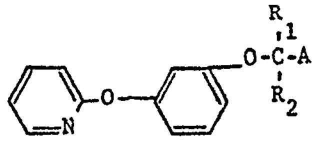

| No. | 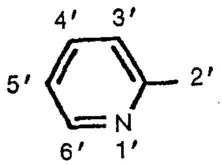 | 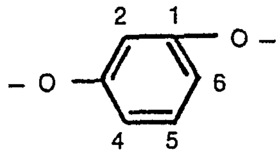 | $-CH(CH_3)-A$<br>A = | Physikalische Konstante |
|---|---|---|---|---|
| 58 | 5'Br | 6 $NO_2$ | $-COSCH_2CH{=}CH_2$ | |
| 59 | 5'Br | 6 $NO_2$ | $-COOCH_2C{\equiv}CH$ | |
| 60 | 3'Cl,5'Cl | 6 Cl | $-COOiC_3H_7$ | $n_D^{25}$ 1.5567 |
| 61 | 3'Cl,5'Cl | 6 Cl | $-COOnC_3H_7$ | |
| 62 | 3'Cl,5'Cl | 6 Cl | $-COOnC_4H_9$ | $n_D^{25}$ 1.5540 |
| 63 | 3'Cl,5'Cl | 6 Cl | $-COOsec.C_4H_9$ | $n_D^{25}$ 1.5525 |
| 64 | 3'Cl,5'Cl | 6 Cl | $-COOtert.C_4H_9$ | |
| 65 | 3'Cl,5'Cl | 6 Cl | $-COOiC_4H_9$ | $n_D^{25}$ 1.5538 |
| 66 | 3'Cl,5'Cl | 6 Cl | $-COOnC_5H_{11}$ | |
| 67 | 3'Cl,5'Cl | 6 Cl | $-COOCH(CH_3)C_3H_7$ | |
| 68 | 3'Cl,5'Cl | 6 Cl | $-COOCH(C_2H_5)_2$ | |
| 69 | 3'Cl,5'Cl | 6 Cl | $-COOnC_6H_{13}$ | |
| 70 | 3'Cl,5'Cl | 6 Cl | $-COOCH(CH_3)C_4H_9$ | |
| 71 | 3'Cl,5'Cl | 6 Cl | $-COOnC_7H_{15}$ | |
| 72 | 3'Cl,5'Cl | 6 Cl | $-COOCH(CH_3)C_5H_{11}$ | |
| 73 | 3'Cl,5'Cl | 6 Cl | $-COOnC_8H_{17}$ | |
| 74 | 3'Cl,5'Cl | 6 Cl | $-COOCH_2CH_2OCH_3$ | $n_D^{25}$ 1.5547 |
| 75 | 3'Cl,5'Cl | 6 Cl | $-COOCH_2CH_2OC_2H_5$ | |
| 76 | 3'Cl,5'Cl | 6 Cl | $-COOCH_2CH_2Cl$ | $n_D^{25}$ 1.5722 |
| 77 | 3'Cl,5'Cl | 6 Cl | $-COOCH_2CH_2Br$ | |
| 78 | 3'Cl,5'Cl | 6 Cl | $-COOCH_2CH{=}CH_2$ | $n_D^{25}$ 1.5659 |
| 79 | 3'Cl,5'Cl | 6 Cl | $-COOCH_2CH{=}CHCH_3$ | |

TABELLE 1 (Fortsetzung)

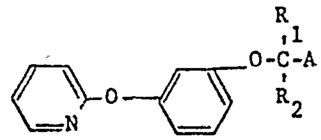

| No. | 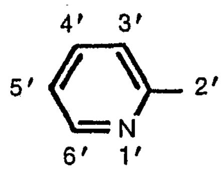 | 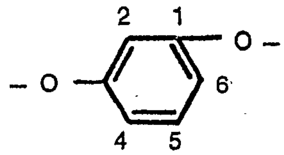 | 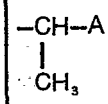 $-CH-A$ / $CH_3$ A = | Physikalische Konstante |
|---|---|---|---|---|
| 80 | 3'Cl,5'Cl | 6 Cl | $-COOCH_2C(CH_3)=CH_2$ | |
| 81 | 3'Cl,5'Cl | 6 Cl | $-COOCH_2C\equiv CH$ | Smp. 64–68° |
| 82 | 3'Cl,5'Cl | 6 Cl | $-COOCH(CH_3)C\equiv CH$ | |
| 83 | 3'Cl,5'Cl | 6 Cl | 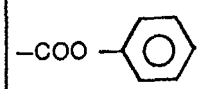 | |
| 84 | 3'Cl,5'Cl | 6 Cl | 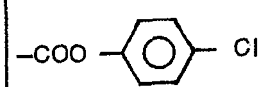 | |
| 85 | 3'Cl,5'Cl | 6 Cl | $-COSCH_3$ | $n_D^{25}$ 1.5850 |
| 86 | 3'Cl,5'Cl | 6 Cl | $-COSC_2H_5$ | |
| 87 | 3'Cl,5'Cl | 6 Cl | $-COSC_3H_7$ | |
| 88 | 3'Cl,5'Cl | 6 Cl | $-COSC_3H_7$(iso) | |
| 89 | 3'Cl,5'Cl | 6 Cl | $-COSC_4H_9$(n) | |
| 90 | 3'Cl,5'Cl | 6 Cl | $-COSCH_2CH(CH_3)_2$ | |
| 91 | 3'Cl,5'Cl | 6 Cl | $-COS$sec.$C_4H_9$ | |
| 92 | 3'Cl,5'Cl | 6 Cl | $-COS$tert.$C_4H_9$ | |
| 93 | 3'Cl,5'Cl | 6 Cl | $-COSC_5H_{11}$ | |
| 94 | 3'Cl,5'Cl | 6 Cl | $-COSCH_2CH=CH_2$ | |
| 95 | 3'Cl,5'Cl | 6 Cl | $-COSCH_2CH=CHCH_3$ | |
| 96 | 3'Cl,5'Cl | 6 Cl | $-COSCH_2COOCH_3$ | |
| 97 | 3'Cl,5'Cl | 6 Cl | –COS-Phenyl | |
| 98 | 3'Cl,5'Cl | 6 Cl | $-COOCH(CH_2)_4$ | $n_D^{30}$ 1.5617 |

TABELLE 1 (Fortsetzung)

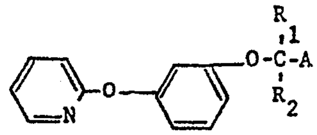

| No. | 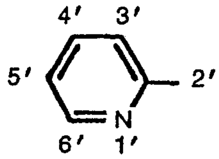 | 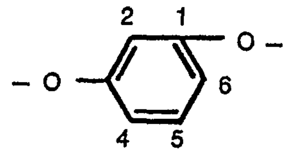 | –CH–A<br>\|<br>$CH_3$<br>A = | Physikalische Konstante |
|---|---|---|---|---|
| 99 | 3'Cl,5'Cl | 6 Cl | $-COOCH(CH_2)_5$ | |
| 100 | 3'Cl,5'Cl | 6 Cl | $-COOCH_2CH(CH_2)_5$ | $n_D^{30}$ 1.5565 |
| 101 | 3'Cl,5'Cl | 6 Cl | $-COOCH_2CH(CH_2)_6$ | |
| 102 | 3'Cl,5'Cl | 6 Cl | $-COOCH_2$ 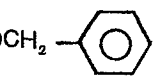 | |
| 103 | 3'Cl,5'Cl | 6 Cl | $-COOCH_2$ 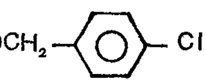 | |
| 104 | 3'Cl,5'Cl | 6 Cl | $-COOCH_2$ 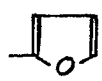 | |
| 105 | 3'Cl,5'Cl | 6 Cl | $-COOCH_2CH-CH_2$ 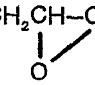 | |
| 106 | 3'Cl,5'Cl | 6 Cl | $-COOCH_2$ 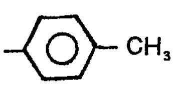 | |
| 107 | 3'Cl,5'Cl | 6 Cl | $-COOCH_2$ 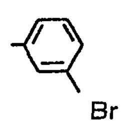 | |
| 108 | 3'Cl,5'Cl | 6 Cl | $-CONHCH_3$ | |

TABELLE 1 (Fortsetzung)

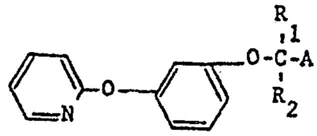

| No. | 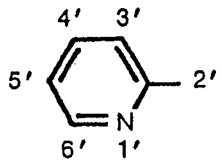 | 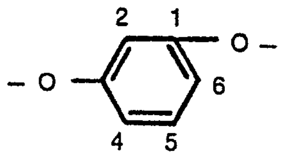 | –CH–A<br>$CH_3$<br>A = | Physikalische Konstante |
|---|---|---|---|---|
| 109 | 3'Cl,5'Cl | 6 Cl | $-CON(CH_3)_2$ | |
| 110 | 3'Cl,5'Cl | 6 Cl | $-CONHC_2H_5$ | |
| 111 | 3'Cl,5'Cl | 6 Cl | $-CONHCH_2CH_2OH$ | |
| 112 | 3'Cl,5'Cl | 6 Cl | $-CONHCH_2CH_2Cl$ | |
| 113 | 3'Cl,5'Cl | 6 Cl | $-CON(C_2H_5)_2$ | Smp. 73–74° |
| 114 | 3'Cl,5'Cl | 6 Cl | $-CONHiC_3H_7$ | |
| 115 | 3'Cl,5'Cl | 6 Cl | $-CONHnC_3H_7$ | |
| 116 | 3'Cl,5'Cl | 6 Cl | $-CONHCH_2CH{=}CH_2$ | Smp. 119–120° |
| 117 | 3'Cl,5'Cl | 6 Cl | $-CON(CH_2CH{=}CH_2)_2$ | |
| 118 | 3'Cl,5'Cl | 6 Cl | $-CONHnC_4H_9$ | |
| 119 | 3'Cl,5'Cl | 6 Cl | $-CONHsec.C_4H_9$ | |
| 120 | 3'Cl,5'Cl | 6 Cl | $-CONHiC_4H_9$ | |
| 121 | 3'Cl,5'Cl | 6 Cl | $-CONHnC_5H_{11}$ | |
| 122 | 3'Cl,5'Cl | 6 Cl | $-CONHCH(CH_3)C_3H_7$ | |
| 123 | 3'Cl,5'Cl | 6 Cl | $-CONH_6H_{13}$ | |
| 124 | 3'Cl,5'Cl | 6 Cl | $-CONHC_7H_{15}$ | |
| 125 | 3'Cl,5'Cl | 6 Cl | $-CONHC_8H_{17}$ | |
| 126 | 3'Cl,5'Cl | 6 Cl | $-COON{=}C(CH_3)_2$ | |
| 127 | 3'Cl,5'Cl | 6 Cl | $-COON{=}C(CH_3)C_2H_5$ | |
| 128 | 3'Cl,5'Cl | 6 Cl | $-COON{=}C(C_2H_5)_2$ | |
| 129 | 3'Cl,5'Cl | 6 Cl | 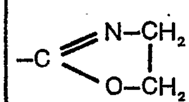 | Smp. 133–135° |
| 130 | 3'Cl,5'Cl | 6 Cl | 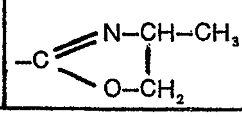 | |

TABELLE 1 (Fortsetzung)

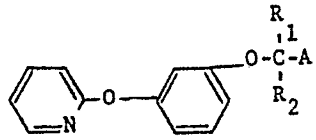

| No. | 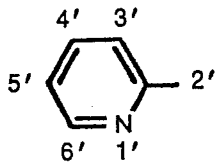 | 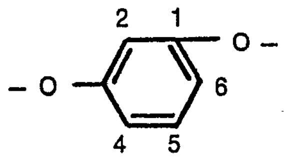 | $-CH(CH_3)-A$<br>A = | Physikalische Konstante |
|---|---|---|---|---|
| 131 | 3'Cl,5'Cl | 6 $NO_2$ | $-COOnC_4H_9$ | |
| 132 | 3'Cl,5'Cl | 6 $NO_2$ | $-COOsec.C_4H_9$ | |
| 133 | 3'Cl,5'Cl | 6 $NO_2$ | $-COOiC_4H_9$ | |
| 134 | 3'Cl,5'Cl | 6 $NO_2$ | $-COOtert.C_4H_9$ | |
| 135 | 3'Cl,5'Cl | 6 $NO_2$ | $-COOnC_5H_{11}$ | |
| 136 | 3'Cl,5'Cl | 6 $NO_2$ | $-COOCH(CH_3)C_3H_7$ | |
| 137 | 3'Cl,5'Cl | 6 $NO_2$ | $-COOCH(C_2H_5)_2$ | |
| 138 | 3'Cl,5'Cl | 6 $NO_2$ | $-COOnC_6H_{13}$ | |
| 139 | 3'Cl,5'Cl | 6 $NO_2$ | $-COOCH(CH_3)C_4H_9$ | |
| 140 | 3'Cl,5'Cl | 6 $NO_2$ | $-COOnC_7H_{15}$ | |
| 141 | 3'Cl,5'Cl | 6 $NO_2$ | $-COOCH(CH_3)C_5H_{11}$ | |
| 142 | 3'Cl,5'Cl | 6 $NO_2$ | $-COOnC_8H_{17}$ | |
| 143 | 3'Cl,5'Cl | 6 $NO_2$ | $-COOCH_2CH_2OCH_3$ | $n_D^{30}$ 1.5709 |
| 144 | 3'Cl,5'Cl | 6 $NO_2$ | $-COOCH_2CH_2OC_2H_5$ | |
| 145 | 3'Cl,5'Cl | 6 $NO_2$ | $-COOCH_2CH_2Cl$ | |
| 146 | 3'Cl,5'Cl | 6 $NO_2$ | $-COOCH_2CH_2Br$ | |
| 147 | 3'Cl,5'Cl | 6 $NO_2$ | $-COOCH_2CH=CH_2$ | $n_D^{30}$ 1.5771 |
| 148 | 3'Cl,5'Cl | 6 $NO_2$ | $-COOCH_2CH=CHCH_3$ | |
| 149 | 3'Cl,5'Cl | 6 $NO_2$ | $-COOCH_2C(CH_3)=CH_2$ | |
| 150 | 3'Cl,5'Cl | 6 $NO_2$ | $-COOCH_2C\equiv CH$ | |
| 151 | 3'Cl,5'Cl | 6 $NO_2$ | $-COOCH(CH_3)C\equiv CH$ | |
| 152 | 3'Cl,5'Cl | 6 $NO_2$ | $-COO$ 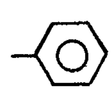 | |

TABELLE 1 (Fortsetzung)

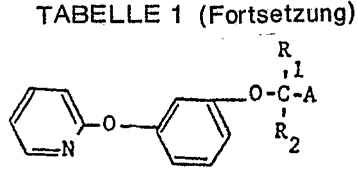

| No. | 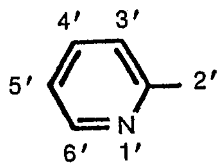 | 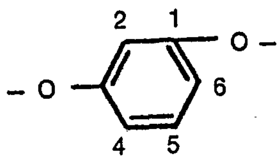 | –CH–A<br>CH$_3$<br>A = | Physikalische Konstante |
|---|---|---|---|---|
| 153 | 3'Cl,5'Cl | 6 $NO_2$ | –COO–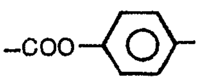–Cl | |
| 154 | 3'Cl,5'Cl | 6 $NO_2$ | $-COSCH_3$ | |
| 155 | 3'Cl,5'Cl | 6 $NO_2$ | $-COSC_2H_5$ | |
| 156 | 3'Cl,5'Cl | 6 $NO_2$ | $-COSC_3H_7$ | |
| 157 | 3'Cl,5'Cl | 6 $NO_2$ | $-COSC_3H_7$iso | |
| 158 | 3'Cl,5'Cl | 6 $NO_2$ | $-COSC_4H_9$n | |
| 159 | 3'Cl,5'Cl | 6 $NO_2$ | $-COSCH_2CH(CH_3)_2$ | |
| 160 | 3'Cl,5'Cl | 6 $NO_2$ | $-COSC_4H_9$sec. | |
| 161 | 3'Cl,5'Cl | 6 $NO_2$ | –COStert.$C_4H_9$ | |
| 162 | 3'Cl,5'Cl | 6 $NO_2$ | $-COSC_5H_{11}$ | |
| 163 | 3'Cl,5'Cl | 6 $NO_2$ | $-COSCH_2CH{=}CH_2$ | |
| 164 | 3'Cl,5'Cl | 6 $NO_2$ | $-COSCH_2CH{=}CH_2CH_3$ | |
| 165 | 3'Cl,5'Cl | 6 $NO_2$ | $-COSCH_2COOCH_3$ | |
| 166 | 3'Cl,5'Cl | 6 $NO_2$ | –COS-Phenyl | |
| 167 | 3'Cl,5'Cl | 6 $NO_2$ | $-COOCH(CH_2)_4$ | |
| 168 | 3'Cl,5'Cl | 6 $NO_2$ | $-COOCH(CH_2)_5$ | |
| 169 | 3'Cl,5'Cl | 6 $NO_2$ | $-COOCH_2CH(CH_2)_5$ | |
| 170 | 3'Cl,5'Cl | 6 $NO_2$ | $-COOCH_2CH(CH_2)_6$ | |
| 171 | 3'Cl,5'Cl | 6 $NO_2$ | $-COOCH_2$–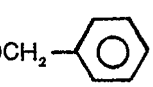 | |

TABELLE 1 (Fortsetzung)

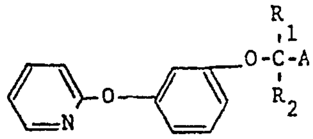

| No. | 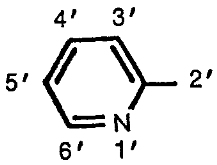 | 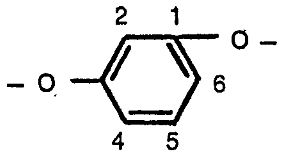 | $-CH(CH_3)-A$<br>A = | Physikalische Konstante |
|---|---|---|---|---|
| 172 | 3'Cl,5'Cl | 6 $NO_2$ | $-COOCH_2-$ 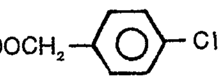 | |
| 173 | 3'Cl,5'Cl | 6 $NO_2$ | $-COOCH_2-$ 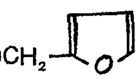 | |
| 174 | 3'Cl,5'Cl | 6 $NO_2$ | $-COOCH_2$ 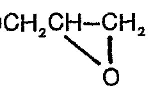 | |
| 175 | 3'Cl,5'Cl | 6 $NO_2$ | $-COOCH_2-$ 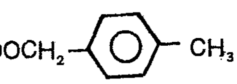 | |
| 176 | 3'Cl,5'Cl | 6 $NO_2$ | $-COOCH_2$ 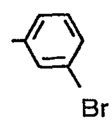 | |
| 177 | 3'Cl,5'Cl | 6 $NO_2$ | $-CONHCH_3$ | |
| 178 | 3'Cl,5'Cl | 6 $NO_2$ | $-CON(CH_3)_2$ | |
| 179 | 3'Cl,5'Cl | 6 $NO_2$ | $-CONHC_2H_5$ | Smp. 128–129° |
| 180 | 3'Cl,5'Cl | 6 $NO_2$ | $-CONHCH_2CH_2OH$ | |
| 181 | 3'Cl,5'Cl | 6 $NO_2$ | $-CONHCH_2CH_2Cl$ | |
| 182 | 3'Cl,5'Cl | 6 $NO_2$ | $-CON(C_2H_5)_2$ | |
| 183 | 3'Cl,5'Cl | 6 $NO_2$ | $-CONHiC_3H_7$ | |

TABELLE 1 (Fortsetzung)

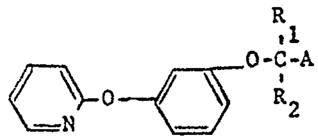

| No. | 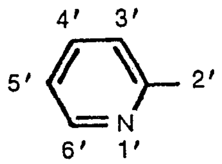 | 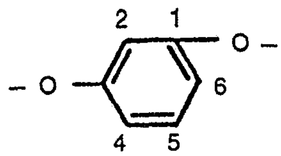 | –CH–A<br>CH$_3$<br>A = | Physikalische Konstante |
|---|---|---|---|---|
| 184 | 3'Cl,5'Cl | 6 $NO_2$ | $-CONHn-C_3H_7$ | |
| 185 | 3'Cl,5'Cl | 6 $NO_2$ | $-CONHCH_2CH=CH_2$ | |
| 186 | 3'Cl,5'Cl | 6 $NO_2$ | $-CON(CH_2CH=CH_2)_2$ | |
| 187 | 3'Cl,5'Cl | 6 $NO_2$ | $-CONHnC_4H_9$ | |
| 188 | 3'Cl,5'Cl | 6 $NO_2$ | $-CONHiC_4H_9$ | |
| 189 | 3'Cl,5'Cl | 6 $NO_2$ | $-CONsec.C_4H_9$ | |
| 190 | 3'Cl,5'Cl | 6 $NO_2$ | $-CONHnC_5H_{11}$ | |
| 191 | 3'Cl,5'Cl | 6 $NO_2$ | $-CONCH(CH_3)C_3H_7$ | |
| 192 | 3'Cl,5'Cl | 6 $NO_2$ | $-CONHC_6H_{13}$ | |
| 193 | 3'Cl,5'Cl | 6 $NO_2$ | $-CONHC_7H_{15}$ | |
| 194 | 3'Cl,5'Cl | 6 $NO_2$ | $-CONHC_8H_{17}$ | |
| 195 | 3'Cl,5'Cl | 6 $NO_2$ | $-COON=C(CH_3)_2$ | |
| 196 | 3'Cl,5'Cl | 6 $NO_2$ | $-CON=C(CH_3)C_2H_5$ | |
| 197 | 3'Cl,5'Cl | 6 $NO_2$ | $-COON=(C_2H_5)_2$ | |
| 198 | 3'Cl,5'Cl | 6 $NO_2$ | 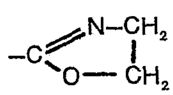 | |
| 199 | 3'Cl,5'Cl | 6 $NO_2$ | 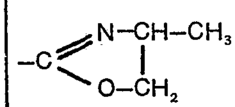 | |
| 200 | 3'Cl,5'Cl | 6 $NO_2$ | $-COOiC_3H_7$ | $n_D^{30}$ 1.5638 |
| 201 | 3'Cl,5'Cl | 6 $NO_2$ | $-COOnC_3H_7$ | |
| 202 | 3'Cl,5'Cl | 6 $NO_2$ | $-CONH_2$ | Smp. 135–137° |
| 203 | 3'Cl,5'Cl | 6 $NO_2$ | $-COOH$ | Smp. 134–136° |
| 204 | 3'Cl,5'Cl | 6 $NO_2$ | $-COS^{\ominus}Na^{\oplus}$ | |
| 205 | 3'Cl,5'Cl | 6 $NO_2$ | –CON 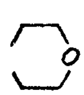 | |

TABELLE 1 (Fortsetzung)

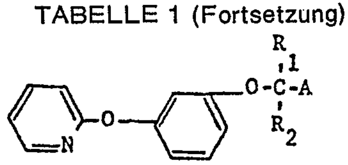

| No. | 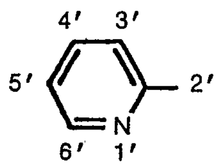 | 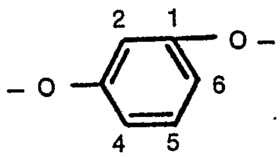 | 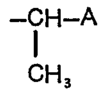 A = | Physikalische Konstante |
|---|---|---|---|---|
| 206 | 3'Cl,5'Cl | 6 $NO_2$ | –CON 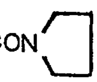 | |
| 207 | 3'Cl,5'Cl | 6 $NO_2$ | –CO 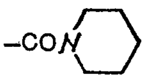 | |
| 208 | 3'Cl,5'Cl | 6 $NO_2$ | $-CONHCH_2CH_2Br$ | |
| 209 | 3'Cl,5'Cl | 6 $NO_2$ | $-CONHCH(C_2H_5)_2$ | |
| 210 | 3'Cl,5'Cl | 6 Cl | $-CONH_2$ | |
| 211 | 3'Cl,5'Cl | 6 Cl | $-COO^{\ominus}Na^{\oplus}$ | Smp. 98–101° |
| 212 | 3'Cl,5'Cl | 6 Cl | 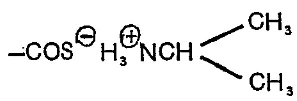 | |
| 213 | 3'Cl,5'Cl | 6 Cl | –CON 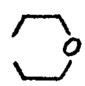 | Smp. 39–42° |
| 214 | 3'Cl,5'Cl | 6 Cl | –CON 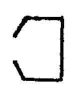 | |
| 215 | 3'Cl,5'Cl | 6 Cl | –CO 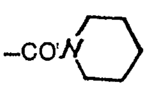 | |
| 216 | 3'Cl,5'Cl | 6 Cl | $-CONHCH_2COOC_2H_5$ | |
| 217 | 3'Cl,5'Cl | 6 Cl | $-COOCH_2CN$ | |

TABELLE 1 (Fortsetzung)

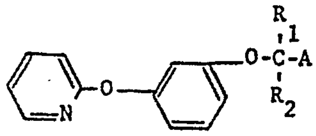

| No. | 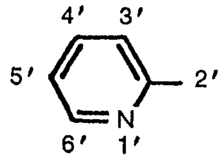 | 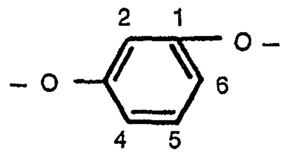 | 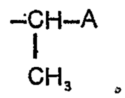 A = | Physikalische Konstante |
|---|---|---|---|---|
| 218 | 3'Cl,5'Cl | 6 Cl | –COO 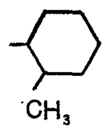 | |
| 219 | 3'Cl,5'Cl | 6 Cl | $-COO^{\ominus}NH_4^{\oplus}$ | |
| 220 | 3'Cl,5'Cl | 6 Cl | $-COOCH_2CH_2OC_4H_9$ | $n_D^{30}$ 1.5445 |
| 221 | 3'Cl,5'Cl | 6 Br | $-COOCH_2$ 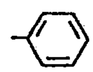 | $n_D^{30}$ 1.5811 |
| 222 | 3'Cl,5'Cl | 6 Br | $-COOC_2H_5$ | |
| 223 | 3'Cl,5'Cl | 6 Br | $-COOiC_3H_7$ | Smp. 60–63° |
| 224 | 3'Cl,5'Cl | 6 Br | $-COOnC_3H_7$ | |
| 225 | 3'Cl,5'Cl | 6 Br | $-COOnC_4H_9$ | |
| 226 | 3'Cl,5'Cl | 6 Br | $-COOsec.C_4H_9$ | |
| 227 | 3'Cl,5'Cl | 6 Br | $-COOtert.C_4H_9$ | |
| 228 | 3'Cl,5'Cl | 6 Br | $-COOiC_4H_9$ | |
| 229 | 3'Cl,5'Cl | 6 Br | $-COOnC_5H_{11}$ | |
| 230 | 3'Cl,5'Cl | 6 Br | $-COOCH(CH_3)C_3H_7$ | $n_D^{30}$ 1.5561 |
| 231 | 3'Cl,5'Cl | 6 Br | $-COOCH(C_2H_5)_2$ | |
| 232 | 3'Cl,5'Cl | 6 Br | $-COOnC_6H_{13}$ | |
| 233 | 3'Cl,5'Cl | 6 Br | $-COOnC_7H_{15}$ | |
| 234 | 3'Cl,5'Cl | 6 Br | $-COOnC_8H_{17}$ | $n_D^{30}$ 1.5458 |

TABELLE 1 (Fortsetzung)

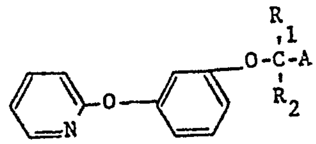

| No. | 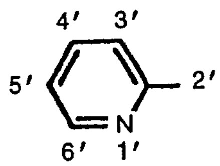 | 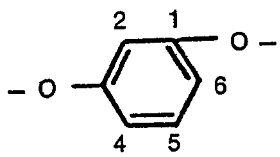 | –CH–A<br>\|<br>$CH_3$<br>A = | Physikalische Konstante |
|---|---|---|---|---|
| 235 | 3'Cl,5'Cl | 6 Br | $-COOCH_2CH_2OCH_3$ | |
| 236 | 3'Cl,5'Cl | 6 Br | $-COOCH_2CH_2OC_2H_5$ | $n_D^{30}$ 1.5631 |
| 237 | 3'Cl,5'Cl | 6 Br | $-COOCH_2CH_2Cl$ | $n_D^{30}$ 1.5792 |
| 238 | 3'Cl,5'Cl | 6 Br | $-COOCH_2CH_2Br$ | |
| 239 | 3'Cl,5'Cl | 6 Br | $-COOCH_2CH{=}CH_2$ | |
| 240 | 3'Cl,5'Cl | 6 Br | $-COOCH_2CH{=}CHCH_3$ | $n_D^{30}$ 1.5730 |
| 241 | 3'Cl,5'Cl | 6 Br | $-COOCH_2C{\equiv}CH$ | Smp. 80–82° |
| 242 | 3'Cl,5'Cl | 6 Br | –COOPhenyl | |
| 243 | 3'Cl,5'Cl | 6 Br | $-COSCH_3$ | |
| 244 | 3'Cl,5'Cl | 6 Br | $-CSOC_2H_5$ | |
| 245 | 3'Cl,5'Cl | 6 Br | $-COSC_3H_7n$ | |
| 246 | 3'Cl,5'Cl | 6 Br | $-COSC_3H_7iso$ | $n_D^{30}$ 1.5795 |
| 247 | 3'Cl,5'Cl | 6 Br | $-COSC_4H_9n$ | |
| 248 | 3'Cl,5'Cl | 6 Br | $-COSCH_2CH(CH_3)_2$ | |
| 249 | 3'Cl,5'Cl | 6 Br | $-COSCH_2CH{=}CH_2$ | |
| 250 | 3'Cl,5'Cl | 6 Br | $-COSCH_2COOCH_3$ | $n_D^{30}$ 1.5780 |
| 251 | 3'Cl,5'Cl | 6 Br | $-CONH_2$ | Smp. 155–156° |
| 252 | 3'Cl,5'Cl | 6 Br | $-CONHCH_3$ | |
| 253 | 3'Cl,5'Cl | 6 Br | $-CON(CH_3)_2$ | |
| 254 | 3'Cl,5'Cl | 6 Br | $-CONHC_2H_5$ | Smp. 114–115° |
| 255 | 3'Cl,5'Cl | 6 Br | $-CON(C_2H_5)_2$ | |
| 256 | 3'Cl,5'Cl | 6 Br | $-CONHnC_3H_7$ | |

TABELLE 1 (Fortsetzung)

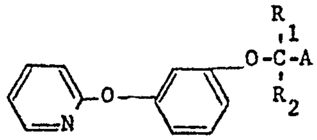

| No. | 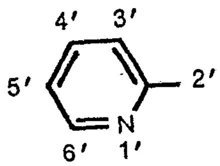 | 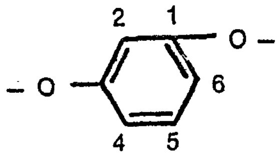 | $-CH(CH_3)-A$<br>A = | Physikalische Konstante |
|---|---|---|---|---|
| 257 | 3'Cl,5'Cl | 6 Br | $-CONHiC_3H_7$ | |
| 258 | 3'Cl,5'Cl | 6 Br | $-CONHnC_4H_9$ | |
| 259 | 3'Cl,5'Cl | 6 Br | $-CONHsec.C_4H_9$ | |
| 260 | 3'Cl,5'Cl | 6 Br | $-CONHiC_4H_9$ | |
| 261 | 3'Cl,5'Cl | 6 Br | $-CONHnC_5H_{11}$ | |
| 262 | 3'Cl,5'Cl | 6 Br | $-CONHnC_6H_{13}$ | |
| 263 | 3'Cl,5'Cl | 6 Br | $-CONHnC_7H_{15}$ | |
| 264 | 3'Cl,5'Cl | 6 Br | $-CONHnC_8H_{17}$ | |
| 265 | 3'Cl,5'Cl | 6 Br | $-CONHCH_2CH=CH_2$ | |
| 266 | 3'Cl,5'Cl | 6 Br | $-CON(CH_2CH=CH_2)_2$ | |
| 267 | 3'Cl,5'Cl | 6 Br | –CON 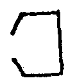 | |
| 268 | 3'Cl,5'Cl | 6 Br | 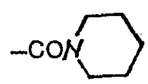 | |
| 269 | 3'Cl,5'Cl | 6 Br | –CON 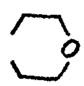 | |
| 270 | 3'Cl,5'Cl | 6 Br | $-CONHCH_2CH_2OH$ | |
| 271 | 3'Cl,5'Cl | 6 Br | $-CONHCH_2CH_2Cl$ | |
| 272 | 3'Cl,5'Cl | 6 Br | 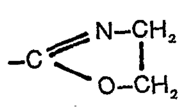 | |
| 273 | 3'Cl,5'Cl | 6 Br | $-COON=C(CH_3)_2$ | |
| 274 | 3'Cl,5'Cl | 6 Br | $-COOCH_2CN$ | |

TABELLE 1 (Fortsetzung)

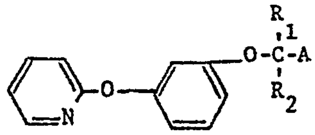

| No. | 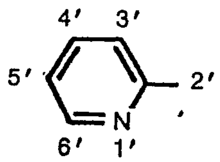 | 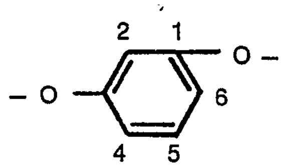 | –CH–A<br>CH$_3$<br>A = | Physikalische Konstante |
|---|---|---|---|---|
| 275 | 3'Br,5'Cl | 6 Cl | $-COOCH_3$ | |
| 276 | 3'Br,5'Cl | 6 Cl | $-COOC_2H_5$ | |
| 277 | 3'Br,5'Cl | 6 Cl | $-COOnC_3H_7$ | |
| 278 | 3'Br,5'Cl | 6 Cl | $-COOiC_3H_7$ | |
| 279 | 3'Br,5'Cl | 6 Cl | $-COOnC_4H_9$ | |
| 280 | 3'Br,5'Cl | 6 Cl | $-COOiC_4H_9$ | |
| 281 | 3'Br,5'Cl | 6 Cl | $-COOsec.C_4H_9$ | |
| 282 | 3'Br,5'Cl | 6 Cl | $-CONH_2$ | |
| 283 | 3'Br,5'Cl | 6 Cl | $-CONHCH_3$ | |
| 284 | 3'Br,5'Cl | 6 Cl | $-CON(CH_3)_2$ | |
| 285 | 3'Br,5'Cl | 6 Cl | $-CONHC_2H_5$ | |
| 286 | 3'Br,5'Cl | 6 Cl | $-CONHnC_3H_7$ | |
| 287 | 3'Br,5'Cl | 6 Cl | $-CONHiC_3H_7$ | |
| 288 | 3'Br,5'Cl | 6 Cl | $-CONHnC_4H_9$ | |
| 289 | 3'Br,5'Cl | 6 Cl | $-CONHsecC_4H_9$ | |
| 290 | 3'Br,5'Cl | 6 Cl | $-CONHiC_4H_9$ | |
| 291 | 3'Br,5'Cl | 6 Cl | $-CONHtert.C_4H_9$ | |
| 292 | 3'Br,5'Cl | 6 Cl | $-COSCH_3$ | |
| 293 | 3'Br,5'Cl | 6 Cl | $-COSC_2H_5$ | |
| 294 | 3'Br,5'Cl | 6 Cl | $-COSC_3H_7n$ | |
| 295 | 3'Br,5'Cl | 6 Cl | $-COSC_4H_9n$ | |
| 296 | 3'Br,5'Cl | 6 Cl | $-COSCH_2CH=CH_2$ | |
| 297 | 3'Br,5'Cl | 6 $NO_2$ | $-COOCH_3$ | Smp. 93–94° |
| 298 | 3'Br,5'Cl | 6 $NO_2$ | $-COOC_2H_5$ | |

TABELLE 1 (Fortsetzung)

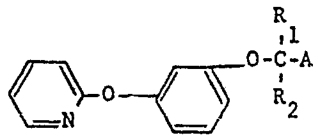

| No. | 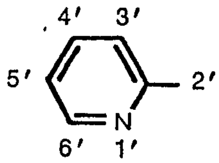 | 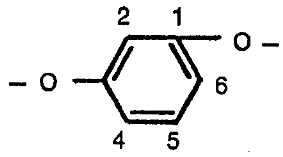 | –CH–A <br> \| <br> $CH_3$ <br> A = | Physikalische Konstante |
|---|---|---|---|---|
| 299 | 3'Br,5'Cl | 6 $NO_2$ | $-COOiC_3H_7$ | |
| 300 | 3'Br,5'Cl | 6 $NO_2$ | $-COSCH_3$ | |
| 301 | 3'Br,5'Cl | 6 $NO_2$ | $-COSC_2H_5$ | |
| 302 | 3'Br,5'Cl | 6 $NO_2$ | $-COSCH_2CH{=}CH_2$ | |
| 303 | 3'Br,5'Cl | 6 $NO_2$ | $-COSCH_2COOCH_3$ | |
| 304 | 3'Cl,5'Br | 6 Cl | $-COOCH_3$ | |
| 305 | 3'Cl,5'Br | 6 Cl | $-COOC_2H_5$ | |
| 306 | 3'Cl,5'Br | 6 Cl | $-COOCH_2CH_2Cl$ | |
| 307 | 3'Cl,5'Br | 6 Cl | $-COOCH_2CH_2OCH_3$ | |
| 308 | 3'Cl,5'Br | 6 Cl | $-COOCH_2CH{=}CH_2$ | |
| 309 | 3'Cl,5'Br | 6 Cl | $-COSCH_3$ | |
| 310 | 3'Cl,5'Br | 6 Cl | $-COSC_2H_5$ | |
| 311 | 3'Cl,5'Br | 6 Cl | $-COSiC_3H_7$ | |
| 312 | 3'Cl,5'Br | 6 Cl | $-COSCH_2CH{=}CH_2$ | |
| 313 | 3'Cl,5'Br | 6 $NO_2$ | $-COOCH_3$ | |
| 314 | 3'Cl,5'Br | 6 $NO_2$ | $-COOC_2H_5$ | |
| 315 | 3'Cl,5'Br | 6 $NO_2$ | $-COOC_3H_7$ | |
| 316 | 3'Cl,5'Br | 6 $NO_2$ | $-COSCH_3$ | |
| 317 | 3'Cl,5'Br | 6 $NO_2$ | $-COSC_2H_5$ | |
| 318 | 3'Cl,5'Br | 6 $NO_2$ | $-CONH_2$ | |
| 319 | 3'Cl,5'Br | 6 $NO_2$ | $-CONHCH_2CH{=}CH_2$ | |
| 320 | 3'Cl,5'Cl | 6 CN | $-COOCH_3$ | |
| 321 | 3'Cl,5'Cl | 6 CN | $-COOC_2H_5$ | |
| 322 | 3'Cl,5'Cl | 6 CN | $-COOC_3H_7$ | |
| 323 | 3'Cl,5'Cl | 6 CN | $-COOCH_2CH_2Cl$ | |

TABELLE 1 (Fortsetzung)

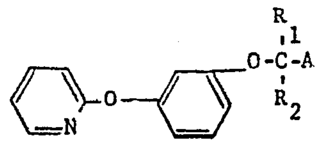

| No. | 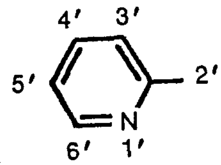 | 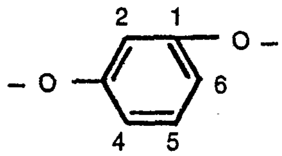 | 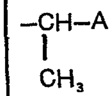 A = | Physikalische Konstante |
|---|---|---|---|---|
| 324 | 3'Cl,5'Cl | 6 CN | $-COOCH_2CH_2OCH_3$ | |
| 325 | 3'Cl,5'Cl | 6 CN | $-COOCH_2CH=CH_2$ | |
| 326 | 3'Cl,5'Cl | 6 CN | $-COOCH_2C\equiv CH$ | |
| 327 | 3'Cl,5'Cl | 6 CN | $-COSCH_3$ | |
| 328 | 3'Cl,5'Cl | 6 CN | $-COSC_2H_5$ | |
| 329 | 3'Cl,5'Cl | 6 CN | $-COSCH_2CH=CH_2$ | |
| 330 | 3'Cl,5'Cl | 6 CN | $-CONHCH_2CH=CH_2$ | |
| 331 | 3'Br,5'Br | – | $-COOCH_3$ | Smp. 58–60° |
| 332 | 3'Br,5'Br | 6 Cl | $-COOCH_3$ | Smp. 86–88° |
| 333 | 3'Br,5'Br | 6 $NO_2$ | $-COOCH_3$ | Smp. 118–120° |
| 334 | 3'Br,5'Br | 4 $NO_2$, 6 $NO_2$ | $-COOCH_3$ | Smp. 174–178° |
| 335 | 3'Cl,5'Cl | 2 $NO_2$ | $-COOCH_3$ | Smp. 104–106° |
| 336 | 3'Cl,5'Cl | 4 $NO_2$, 6 $NO_2$ | $-COOCH_3$ | Smp. 159–163° |
| 337 | 3'Cl,5'Cl | 4 $NO_2$ | $-COOCH_3$ | Smp. 88–89° |
| 338 | 3'Cl,5'Cl | 4 Cl, 6 $NO_2$ | $-COOCH_3$ | |

TABELLE 1 (Fortsetzung)

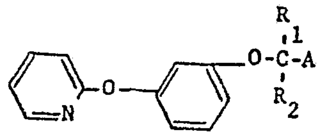

| No. | 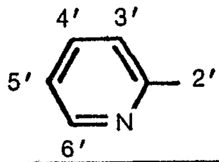 | 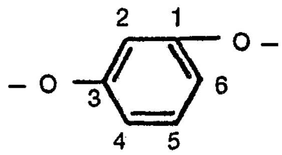 | 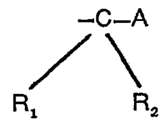 | Physikalische Konstante |
|---|---|---|---|---|
| 339 | 3'Cl,5'Cl | 6 $NO_2$ | $-CH_2CN$ | Smp. 118–120° |
| 340 | 3'Cl,5'Cl | 6 Br | $-CH_2COOCH_3$ | Smp. 81–82° |
| 341 | 3'Cl,5'Cl | 4 Br | $CH_3$<br>$-CHCOOCH_3$ | Smp. 103–104° |
| 342 | 3'Cl,5'Cl | 6 Br | $CH_3$<br>$-CHCOOH$ | Smp. 150–151° |
| 343 | 3'Cl,5'CN,6'$CH_3$ | 6 Cl | $CH_3$<br>$-CHCOOCH_3$ | Smp. 108–109° |
| 344 | 5'CN,6'Cl | 6 Cl | $CH_3$<br>$-CHCOOCH_3$ | |
| 345 | 5'Cl,6'$CF_3$ | 6 Cl | $CH_3$<br>$-CHCOOCH_3$ | Smp. 92–94° |
| 346 | 5'$NO_2$ | 6 Cl | $CH_3$<br>$-CHCOOCH_3$ | |
| 347 | 5'$CF_3$,6'Cl | 6 Cl | $CH_3$<br>$-CHCOOCH_3$ | Smp. 71–72° |
| 348 | 3'Cl,5'Cl | 6 Cl | $CH_3$<br>$-CHCONH(CH_2)_2OCH_3$ | |
| 349 | 3'Cl,5'Cl | 6 $NO_2$ | $CH_3$<br>$-CHCONH(CH_2)_2OC_2H_5$ | |
| 350 | 3'Cl,5'Cl | 6 $NO_2$ | $CH_3$<br>$-CHCONH(CH_2)_3OCH_3$ | |
| 351 | 3'Cl,5'Cl | 6 $NO_2$ | $CH_3$<br>$-CHCONH(CH_2)_3OC_2H_5$ | |

TABELLE 2

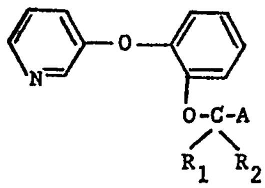

| No. | 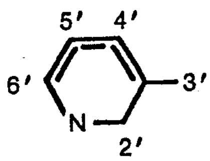 | 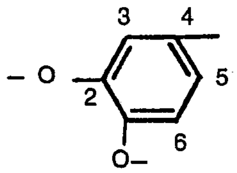 | 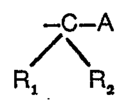 | Physikalische Konstante |
|---|---|---|---|---|
| 1 | 2'Cl,6'Cl | – | $CH_3$<br>\|<br>$-CHCOOCH_3$ | |
| 2 | 2'$CH_3$,6'$CH_3$ | – | $CH_3$<br>\|<br>$-CHCOOCH_3$ | |
| 3 | 2'Cl,4'Cl,6'$CH_3$ | – | $CH_3$<br>\|<br>$-CHCOOCH_3$ | |
| 4 | 2'Cl,6'Cl | 4 Cl | $CH_3$<br>\|<br>$-CHCOOCH_3$ | |

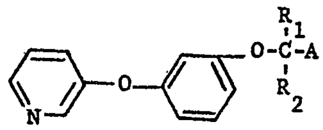

TABELLE 3

| No. | 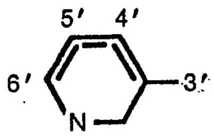 | 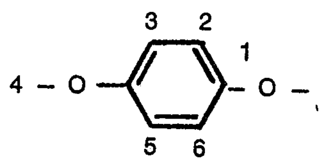 | $R_1$<br>\|<br>–C–A<br>\|<br>$R_2$ | Physikalische Konstante |
|---|---|---|---|---|
| 1 | 2'Cl,6'Cl | – | $CH_3$<br>\|<br>$-CHCOOCH_3$ | Smp. 72° |
| 2 | 2'Cl,6'Br | – | $CH_3$<br>\|<br>$-CHCOOC_2H_5$ | |
| 3 | 2'$CH_3$,6'$CH_3$ | – | $CH_3$<br>\|<br>$-CHCOOCH_3$ | $n_D^{25}$ 1.5426 |
| 4 | 2'Cl,4'Cl,6$CH_3$ | – | $CH_3$<br>\|<br>$-CHCOOCH_3$ | |
| 5 | 2'Cl,6'CN | – | $CH_3$<br>\|<br>$-CHCOOCH_3$ | |
| 6 | 2'Cl,6'Cl | – | $-CH_2COOC_2H_5$ | Smp. 73° |
| 7 | 2'Cl,6'Cl | – | $CH_3$<br>\|<br>$-CHCOOCH_2$ – 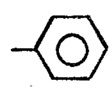 | |
| 9 | 2'Cl,6'Cl | – | $CH_3$<br>\|<br>$-CHCOON{=}C(CH_3)_2$ | |
| 10 | 2'Cl,6'Cl | – | $CH_3$<br>\|<br>$-CHCOOC_2H_4Cl$ | |
| 11 | 2'Cl,6'Cl | – | $CH_3$<br>\|<br>$-CHCON(CH_3)_2$ | |
| 12 | 2'Cl,6'Cl | 2 Cl | $CH_3$<br>\|<br>$-CHCOOCH_3$ | |

TABELLE 3 (Fortsetzung)

| No. | 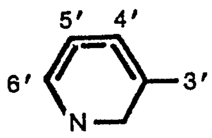 | 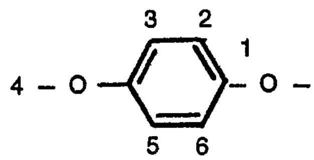 | $R_1$<br>$-C-A$<br>$R_2$ | Physikalische Konstante |
|---|---|---|---|---|
| 13 | 2'Cl,6'Cl | – | $CH_2OCH_3$<br>$-CHCOOCH_3$ | |
| 14 | 2'Cl,6'Cl | – | $CH_3$<br>$-CHCOSCH_3$ | |
| 15 | 6'$CF_3$ | | $CH_3$<br>$-CHCOOCH_3$ | $n_D^{30}$ 1.5080 |

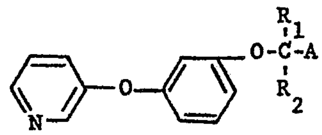

TABELLE 4

| No. | 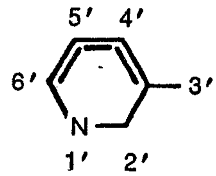 | 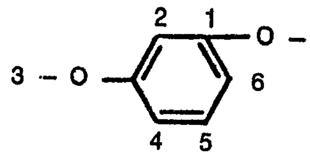 | $R_1'$<br>\|<br>–C–A<br>\|<br>$R_2$ | Physikalische Konstante |
|---|---|---|---|---|
| 1 | 2'Cl,6'Cl | 6 $NO_2$ | $CH_3$<br>\|<br>$-CHCOOCH_3$ | |
| 2 | 2'Cl,6'Cl | 6 Cl | $CH_3$<br>\|<br>$-CHCOOCH_3$ | |
| 3 | 2'Cl,6'Cl | 6 CN | $CH_3$<br>\|<br>$-CHCOOCH_3$ | |
| 4 | 2'$CH_3$,6'$CH_3$ | 6 $NO_2$ | $CH_3$<br>\|<br>$-CHCOOCH_3$ | |
| 5 | 2'$CH_3$,6'$CH_3$ | 6 Cl | $CH_3$<br>\|<br>$-CHCOOCH_3$ | |
| 6 | 2'$CH_3$,6'$CH_3$ | 6 CN | $CH_3$<br>\|<br>$-CHCOOCH_3$ | |
| 7 | 2'Cl,6'Cl | 6 $CSNH_2$ | $CH_3$<br>\|<br>$-CHCOOCH_3$ | |
| 8 | 2'Cl,6'Br | 6 $NO_2$ | $CH_3$<br>\|<br>$-CHCOOCH_3$ | |
| 9 | 2'Cl,6'Br | 6 Cl | $CH_3$<br>\|<br>$-CHCOOCH_3$ | |
| 10 | 2'Cl,4'Cl,6'$CH_3$ | 6 $NO_2$ | $CH_3$<br>\|<br>$-CHCOOCH_3$ | |
| 11 | 2'Cl,4'Cl,6'$CH_3$ | 6 Cl | $CH_3$<br>\|<br>$-COOCH_3$ | |

TABELLE 4 (Fortsetzung)

| No. | 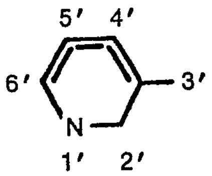 | 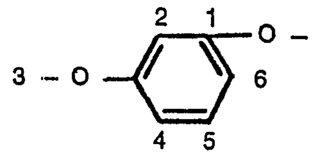 | $R_1$<br>\|<br>$-C-A$<br>\|<br>$R_2$ | Physikalische Konstante |
|---|---|---|---|---|
| 12 | 2'Cl, 5'Cl, 6'Cl | – | $CH_3$<br>\|<br>$-CHCOOCH_3$ | |
| 13 | 2'Cl, 5'Cl, 6'Cl | 6 $NO_2$ | $CH_3$<br>\|<br>$-CHCOOCH_3$ | |
| 14 | 2'Cl, 5'Cl, 6'Cl | 6 Cl | $CH_3$<br>\|<br>$-CHCOOCH_3$ | |

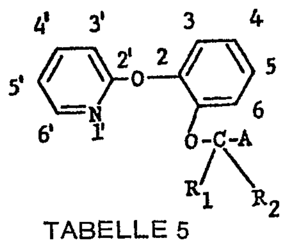

TABELLE 5

| No. | 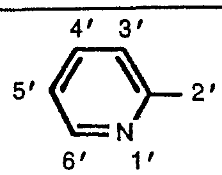 | 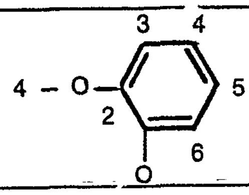 | 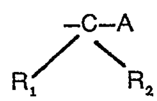 | Physikalische Konstante |
|---|---|---|---|---|
| 1 | 3'Cl, 5'Cl | – | $CH_3$<br>\|<br>$-CHCOOCH_3$ | Smp. 69–72° |
| 2 | 3'Cl, 5'CN, 6'$CH_3$ | – | $CH_3$<br>\|<br>$-CHCOOCH_3$ | |
| 3 | 6'Cl | – | $CH_3$<br>\|<br>$-CHCOOCH_3$ | |
| 4 | 3'Cl, 5'Cl | 4 Cl | $CH_3$<br>\|<br>$-CHCOOC_2H_4OCH_3$ | |
| 5 | 5'Cl, 5'Cl | 4 Cl, 6 Cl | $CH_3$<br>\|<br>$-CHCOOCH_3$ | |
| 6 | 3'Cl, 5'Cl | 4 $CH_3$ | $CH_3$<br>\|<br>$-CHCOOCH_3$ | |
| 7 | 3'Cl, 5'CN | – | $CH_3$<br>\|<br>$-CHCOOCH_3$ | |

Die Herstellung erfindungsgemässer Mittel erfolgt in an sich bekannter Weise durch inniges Vermischen und Vermahlen von Wirkstoffen der Formel I mit geeigneten Trägerstoffen, gegebenenfalls unter Zusatz von gegenüber den Wirkstoffen inerten Dispersions- oder Lösungsmitteln. Die Wirkstoffe können in den folgenden Aufarbeitungsformen vorliegen und angewendet werden:

feste Aufarbeitungsformen:
Stäubemittel, Streumittel, Granulate, Umhüllungsgranulate, Imprägnierungsgranulate und Homogengranulate;
in Wasser dispergierbare Wirkstoffkonzentrate:
Spritzpulver, (wettable powder)
Pasten, Emulsionen:
flüssige Aufarbeitungsformen:
Lösungen.

Der Gehalt an Wirkstoff in den oben beschriebenen Mitteln liegt zwischen 0,1 bis 95%, bevorzugt zwischen 1 bis 80%. Anwendungsformen können bis hinab zu 0,001% verdünnt werden. Die Aufwandmengen betragen in der Regel 0,1 bis 10 kg AS/ha, vorzugsweise 0,25 bis 5 kg AS/ha. Den beschriebenen erfindungsgemässen Mitteln lassen sich auch andere biozide Wirkstoffe oder Mittel beimischen. Die Wirkstoffe der Formel I können beispielsweise wie folgt formuliert werden (Teile bedeuten Gewichtsteile):

*Stäubemittel*

Zur Herstellung eines a) 5%igen und b) 2%igen Stäubemittels werden die folgenden Stoffe verwendet:

a) 5 Teile $\alpha$ - [3 - 3',5' - Dichlorpyridyl - 2' - oxy) - 6 - chlorphenoxy] - propionsäure-methylester,
95 Teile Talkum,
b) 2 Teile des obigen Wirkstoffes,
1 Teil hochdisperse Kieselräure,
97 Teile Talkum;

Die Wirkstoffe werden mit den Trägerstoffen vermischt und vermahlen.

*Granulat*

Zur Herstellung eines 5%igen Granulates werden die folgenden Stoffe verwendet:

5 Teile des obigen Wirkstoffes,
0,25 Teile Epichlorhydrin
0,25 Teile Cetylpolyäthylenglykoläther mit 8 Mol Aethylenoxyd,
3,50 Teile Polyäthylenglykol,
91 Teile Kaolin (Korngrösse 0,3 bis 0,8 mm)

Die Aktivsubstanz wird mit Epichlorhydrin vermischt und in 6 Teilen Aceton gelöst, hierauf werden Polyäthylenglykol und Cetylpolyäthylenglykoläther zugesetzt. Die so erhaltene Lösung wird auf Kaolin aufgesprüht und anschliessend im Vakuum eingedampft.

*Spritzpulver*

Zur Herstellung eines a) 50%igen, b) 25%igen und c) 10%igen Spritzpulvers werden folgende Bestandteile verwendet:

a) 50 Teile $\alpha$ - [3 - (3',5' - Dichlorpyridyl - 2' - oxy) - 6 - bromophenoxy] - propionsäure-methylester,
5 Teile Natriumdibutylnaphthylsulfonat,
3 Teile Naphthalinsulfonsäuren - Phenolsulfonsäuren - Formaldehyd - Kondensat 3:2:1,
20 Teile Kaolin,
22 Teile Champagne-Kreide;
b) 25 Teile des obigen Wirkstoffes,
5 Teile Oleylmethyltaurid-Natrium-Salz,
2,5 Teile Naphthalinsulfonsäuren-Formaldehyd-Kondensat,
0,5 Teile Carboxymethylcellulose,
5 Teile neutrales Kalium-Aluminium-Silikat,
62 Teile Kaolin;
c) 10 Teile des obigen Wirkstoffs,
3 Teile Gemisch der Natriumsalze von gesättigten Fettalkoholsulfaten,
5 Teile Napthalinsulfonsäuren-Formaldehyd-Kondensat,
82 Teile Kaolin.

Der angegebene Wirkstoff wird auf die entsprechenden Trägerstoffe (Kaolin und Kreide) aufgezogen und anschliessend vermischt und vermahlen. Man erhält Spritzpulver von vorzüglicher Benetzbarkeit und Schwebefähigkeit. Aus solchen Spritzpulvern können durch Verdünnen mit Wasser Suspensionen jeder gewünschten Wirkstoffkonzentration erhalten werden. Derartige Suspensionen werden zur Bekämpfung von Unkräutern und Ungräsern in Kulturpflanzungen in Vorauflaufverfahren und zur Behandlung von Rasenanlagen verwendet.

*Paste*

Zur Herstellung einer 45%igen Paste werden folgende Stoffe verwendet:

45 Teile $\alpha$-[3-(3',5'-Dichlorpyridyl-2'-oxy)-6-nitrophenoxy]-propionsäure-methylester,
5 Teile Natriumaluminiumsilikat,
14 Teile Cetylpolyäthylenglykoläther mit 8 Mol Aethylenoxid,
1 Teil Oleylpolyäthylenglykoläther mit 5 Mol Aethylenoxid,
2 Teile Spindelöl,
23 Teile Wasser,
10 Teile Polyäthylenglykol.

Der Wirkstoff wird mit den Zuschlagstoffen in dazu geigneten Geräten innig vermischt und vermahlen. Man erhält eine Paste, aus der sich durch Verdünnen mit Wasser Suspensionen jeder gewünschten Konzentration herstellen lassen. Die Suspensionen eignen sich zur Behandlung von Rasenanlagen.

*Emulsionskonzentrat*

Zur Herstellung eines 25%igen Emulsionskonzentrates werden

25 Teile $\alpha$-[3-(3',5'-Dichlorpyridyl-2'-oxy)-6-chlorphenoxy]-propionsäure-isopropylester,
5 Teile Mischung von Nonylphenolpolyoxyäthylen und Calcium-dodecylbenzol-sulfonat,
15 Teile Cyclohexan,
55 Teile Xylol

miteinander vermischt. Dieses Konzentrat kann mit Wasser zu Emulsionen auf geeignete Konzentrationen verdünnt werden.

Anstatt des jeweiligen in den vorhergehenden Formulierungsbeispielen angegebenen Wirkstoffs kann auch eine andere der von der Formel I umfassten Verbindungen verwendet werden.

Die in den erfindungsgemässen Mitteln enthaltenen Wirkstoffe beeinflussen das Pflanzenwachstum in verschiedener Weise. So hemmen, verzögern oder unterbinden sie in erster Linie das Wachstum und die Keimung. Es handelt sich dabei also sowohl um pre- und post-emergente Herbizidwirkung als auch um Wuchshemmung.

Erfindungsgemässe Mittel, die als aktive Komponente mindestens eine Verbindung der Formel I enthalten, eignen sich besonders zur Hemmung Kontrolle des Pflanzenwachstums von monocotylen und dicotylen Pflanzen, wie Gräsern, Sträuchern, Bäumen, Getreide- und Leguminosenkulturen, Zuckerrohr, Tabak, Soja, Zwiebel- und Kartoffelknollen, Zierpflanzen, Obstbäumen und Reben.

Die von den Wirkstoffen der Formel I in erster Linie erzielte Wirkung besteht in der gewünschten Reduktion der Pflanzengrösse, insbesondere der Wuchshöhe. Im allgemeinen ist damit eine gewisse Aenderung der Pflanzenform verbunden. In unmittelbarem Zusammenhang zur Verminderung der Wuchshöhe erfährt die Pflanze eine Festigung. Blätter und Stengel sind kräftiger ausgebildet. Durch Verkürzung der Internodienabstände an monocotylen Pflanzen wird die Knickfestigkeit erhöht. Auf diese Weise können Ernteausfälle durch Gewittersturm, Dauerregen, usw., die normalerweise zu einem Lagern von Getreide- und Leguminosenkulturen führen, weitgehend verhindert und damit die Erntearbeit erleichtert werden. Als Nebeneffekt führt verminderte Wuchshöhe bei Nutzpflanzen zu einer Einsparung an Düngemitteln. In gleichem Masse gilt dies auch für Zierpflanzen, Zierrasen, Sportrasen oder sonstige Grünanpflanzungen.

Eines der wichtigsten Probleme an reinen Grasbepflanzungen ist jedoch der Grasschnitt selbst, sei es an Grünanlagen in Wohngegenden, auf Industriegeländen, auf Sportplätzen, an Autostrassen, Flugpisten, Eisenbahndämmen oder Uferböschungen von Gewässern. In all diesen Fällen ist ein periodisches Schneiden des Rasens bzw. des Graswuchses notwendig. Dies ist nicht nur im Hinblick auf Arbeitskräfte und Maschinen sehr aufwendig, sondern bringt im Verkehrsbereich auch erhebliche Gefahren für das betroffene Personal und die Verkehrsteilnehmer mit sich.

Es besteht daher gerade in Gebieten mit grossen Verkehrsnetzen ein dringendes Bedürfnis, die im Hinblick auf die Verfestigung von Seitenstreifen und Böschungen an Verkehrswegen notwendige Grasnarbe einerseits zu erhalten und zu pflegen, andererseits aber mit einfachen Massnahmen während der gesamten Vegetationsperiode auf einer mittleren Wuchshöhe zu halten. Dies wird durch Applikation erfindungsgemässer Wirkstoffe der Formel I auf sehr günstige Weise erreicht.

In analoger Weise kann durch Behandlung von Bäumen, Sträuchern und Hecken, vort allem in

Wohn- und Industriegebieten, mit erfindungsgemässen Verbindungen der Formel I die arbeitsaufwendige Schnitterarbeit rerduziert werden.

Durch den Einsatz erfindungsgemässer Wirkstoffe der Formel I können auch das Triebwachstum und/oder die Fruchtbarkeit von Obstbäumen und Reben vorteilhaft beeinflusst werden.

Zierpflanzen mit starkem Längenwachstum können durch Behandlung mit erfindungsgemässen Wirkstoffen als kompakte Topfpflanzen gezogen werden.

Die Wirkstoffe der Formel I finden auch Anwendung zur Hemmung des Wachstums unerwünschter Geiztriebe, z.B. bei Tabak und Zierpflanzen, wodurch das arbeitsintensive Ausbrechen dieser Triebe von Hand vermieden wird, ferner zur Austriebhemmung bei lagernden Knollen, beispielsweise bei Zierpflanzenknollen, bei Zwiebeln und Kartoffeln, und schliesslich zur Ertragssteigerung bei stark vegetativ wachsenden Kulturpflanzen, wie Soja und Zuckerrohr, indem durch Applikation erfindungsgemässer Wirkstoffe der Uebgergang von der vegetativen zur generativen Wachstumsphase beschleunigt wird.

Bevorzugt setzt man die erfindungsgemässen Wirkstoffe der Formel I zur Wachstumshemmung an Gräsern, Getreidekulturen, Tabak, Soja und Zierpflanzen ein.

Die Aufwandmengen sind verschieden und von Applikationszeitpunkt abhängig. Sie liegen im allgemeinen zwischen 0,1 und 5 kg Wirkstoff pro Hektar, bei Applikation vor dem Auflaufen der Pflanzen und für die Behandlung von bestehenden Kulturen vorzugsweise bis zu 4 kg pro Hektar.

Grosse wirtschaftliche Bedeutung hat die Erleichterung der Fruchtabszission bei der mechanischen und manuellen Ernte von Oliven und Citrusfrüchten gewonnen. Blattabszissionswirkung und Defoliation ist bei der Baumwollernte von Bedeutung.

Die Entfaltung der Wirkung der erfindungsgemässen Wirkstoffe erfolgt sowohl über die oberirdischen Pflanzenteilte (Kontaktwirkung), insbesondere die Blätter, als auch über den Boden, als preemergentes Herbizid (Keimhemmung).

Die Wirkung als starke Wachstumshemmer zeigt sich darin, dass die meisten post-emergent behandelten Pflanzenarten nach dreiwöchiger Versuchsdauer einen Wachstumsstillstand zeigen, wobei die behandelten Pflanzenteile eine dunkelgrüne Färbung annehmen. Die Blätter fallen aber nicht ab.

Diese Wuchshemmung tritt bei einigen Pflanzenarten schon bei einer Dosierung von 0,5 kg/ha und darunter auf.

Da nicht alle Pflanzenarten gleich stark gehemmt werden, ist bei Wahl einer bestimmten niederen Dosierung ein selektiver Einsatz möglich.

Die erfindungsgemässen Wirkstoffe sind auch interessant Kombinationspartner für eine Reihe von Herbiziden der Phenylharnstoff- und Triazinreihe in Getreidekulturen, Mais, Zuckerrohr bezw. im Obst- und Weinbau.

In Gebiet mit erhöhter Erosionsgefahr können die erfindungsgemässen Wirkstoffe als Wuchshemmer in den verschiedensten Kulturen eingesetzt werden.

Dabei wird die Unkrautdecke nicht beseitigt, sondern nur so stark gehemmt, dass keine Konkurrenzierung der Kulturpflanzen mehr auftritt.

Die Wirkstoffe der Formel I zeichnen sich überdies durch eine sehr starke pre-emergente Herbizidwirkung aus, sind also auch ausgeprägte Keimungshemmer.

Zum Nachweis der Brauchbarkeit als Herbizide (pre- und post-emergent) und als Wuchshemmer dienten folgende Testmethoden:

*Pre-emergence Herbizid-Wirkung* (Keimungshemmung)

Im Gewächshaus wird unmittelbar nach der Einsaat der Versuchspflanzen in Saatschalen die Erdoberfläche mit einer wässerigen Suspension der Wirkstoffe, erhalten aus einem 25%-igen Spritzpulver, behandelt. Es wurden vier verschiedene Konzentrationsreihen angewendet, entsprechend 4, 2, 1 und 0,5 kg Wirksubstanz pro Hektar. Die Saatschalen werden im Gewächshaus bei 22—25°C und 50—70% rel. Luftfeuchtigkeit gehalten und der Versuch nach 3 Wochen ausgewertet und die Resultate nach folgender Notenskala bonitiert:

1 = Pflanzen nicht gekeimt oder total abgestorben
2-8 = Zwischenstufen der Schädigung
9 = Pflanzen ungeschädigt (wie unbehandelte Kontrolle).

*Post-emergente Herbizid-Wirkung* (Kontakherbizid)

Eine grössere Anzahl (mindestens 7) Unkräuter und Kulturpflanzen, sowohl monocotyle wie dicotyle, wurden nach den Auflaufen (im 4 - bis - 6 - Blattstadium) mit einer wässerigen Wirkstoffemulsion in Dosierungen von 0,5; 1; 2 und 4 kg Wirksubstanz pro Hektar auf die Pflanzen gespritzt und diese bei 24—26°C und 45—60% rel. Luftfeuchtigkeit gehalten. 5 Tage und 15 Tage nach Behandlung wird der Versuch ausgewertet und das Ergebnis wie im pre-emergent-Versuch nach derselben Notenskala bonitiert.

Die geprüften Verbindungen gemäss der Erfindung zeigten auf einigen Pflanzen ausgeprägte kontaktherbizide Wirkung und auf vielen Pflanzen Wachstumsstillstand als Symptom der wachstumshemmenden Eigenschaften.

*Wuchshemmung bei Gräsern*

In Kunststoffschalen mit Erde-Torf-Sand-Gemisch (6:3:1) wurden Samen der Gräser Lolium perenne, Poa pratensis, Festuca ovina und Dactylis glomerata ausgesät und normal bewässert. Die aufgelaufenen Gräser wurden wöchentlich bis auf 4 cm Höhe zurückgeschnitten und 40 Tage nach der Aussaat und 1 Tag nach dem letzten Schnitt mit wässerigen Spritzbrühen eines Wirkstoffs der Formel I bespritzt. Die Wirkstoffmenge betrug umgerechnet 5 kg Aktivsubstanz pro Hektar. 10 und 21 Tage nach Applikation wurde das Wachstum der Gräser beurteilt.

*Wuchshemmung bei Getreide*

In Kunststoffbechern wurde Sommerweizen (Triticum aestivum), Sommergerste (Hordeum vulgare) und Roggen (Secale) in sterilisierter Erde angesät und im Gewächshaus gezogen. Die Getreidesprösslinge werden 5 Tage nach Aussaat mit einer Spritzbrühe des Wirkstoffs behandelt. Die Blattapplikation entsprach 6 kg Wirkstoff pro Hektar. Die Auswertung erfolgt nach 21 Tage.

Die erfindungsgemässen Wirkstoffe bewirken eine merkliche Wuchshemmung sowohl bei den Gräsern wie beim Getreide.

**Patentansprüche**

1. Pyridyloxy-phenoxy-alkancarbonsäurederivate der Formel I

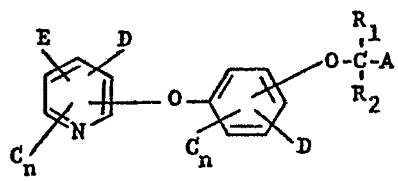

(I)

worin

A die Cyanogruppe, einen Rest —COB oder einen gegebenenfalls durch Methyl ein- oder mehrfach substituierten 2-Oxazolinrest,

B einen Rest $—OR_3$, $—SR_4$ oder $—NR_5R_6$,

C je Wasserstoff Halogen, $C_1—C_4$-Alkyl oder Nitro,

D je Wasserstoff, dasselbe wie C, $C_1—C_4$-Alkoxy, $C_1—C_4$-Alkylthio, Cyano, $C_1—C_4$-Alkylsulfinyl, $C_1—C_4$-Alkylsulfonyl, Thiocarbamid ($—CSNH_2$), Mono- oder Di- $C_1—C_4$-alkylsulfamoyl,

E dasselbe wie C, Mono- oder Di-$C_1—C_4$-Alkylamin, $C_1—C_5$-Alkoxycarbonyl, $C_2—C_8$-Alkoxyalkyl, Cyano, Trifluoromethyl,

n 0, 1 oder 2

$R_1$ Wasserstoff, $C_1—C_4$-Alkyl, $C_2—C_8$-Alkoxyalkyl, $C_1—C_4$-Haloalkyl,

$R_2$ Wasserstoff, $C_1—C_4$-Alkyl, Carbonyl, $C_1—C_5$-Alkoxycarbonyl,

$R_3$ Wasserstoff oder

das Kation einer Base $\frac{1}{m} — M^{m\oplus}$,

wobei

M ein Alkali-, Erdalkali-Kation oder ein Fe-, Cu-, Zn-, 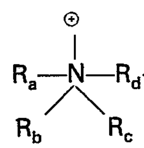 -Kation,

m als ganze Zahl 1, 2 oder 3 die Wertigkeit des Kations berücksichtigt, während $R_a$, $R_b$, $R_c$ und $R_d$ unabhängig voneinander Wasserstoff, Benzyl oder einen gegebenenfalls durch —OH, $—NH_2$ oder $C_1—C_4$-Alkoxy substituierten $C_1—C_4$-Alkylrest bedeuten.

Weiter bedeutet

$R_3$ einen $C_1—C_{18}$-Alkyl-Rest, der unsubstituiert oder gegebenenfalls substituiert ist durch Halogen, Nitro, Cyano, $C_1—C_8$-Alkoxy, $C_2—C_8$-Alkoxyalkoxy, $C_3—C_6$-Alkenyloxy, $C_1—C_8$-Alkylthio, $C_2—C_8$-Alkanoyl, $C_2—C_8$-Acyloxy, $C_2—C_8$-Alkoxycarbonyl, Carbamoyl, Bis-($C_1—C_4$-alkyl)-amino, Tris-($C_1—C_4$-alkyl)-ammonio, $C_3—C_8$-Cycloalkyl, $C_3—C_8$-Cycloalkenyl, gegebenenfalls auch durch einen unsubstituierten oder seinerseits durch Halogen, $C_1—C_4$-Alkyl, $C_1—C_4$-Alkoxy, ein- oder mehrfach substituierten Phenyl-, Phenoxy-, Oxiranyl-, Piperidyl-, Morpholyl-, Pyrrolidinyl- oder Furylrest,

— einen unsubstituierten oder ein- bis vierfach durch Halogen oder einen durch Phenyl oder Methoxycarbonyl substituierten $C_3—C_{10}$-Alkenylrest,

— einen $C_3—C_8$-Alkinyl-Rest,

— einen gegebenenfalls durch Halogen oder $C_1$—$C_4$-Alkyl substituierten $C_3$—$C_{12}$-Cycloalkyl-Rest,
— einen $C_3$—$C_8$-Cycloalkylen-Rest,
— einen Phenylrest, der unsubstituiert oder durch Halogen, $C_1$—$C_4$-Alkyl, $C_1$—$C_4$-Alkoxy, $C_1$—$C_4$-Alkylthio, —$NO_2$, —$CF_3$, —COOH, —CN, —OH, —$SO_3H$, —$NH_2$ oder —NH($C_1$—$C_4$-Alkyl) oder —N($C_1$—$C_4$-Alkyl)$_2$ ein- oder mehrfach substituiert ist,

weiter bedeutet $R_3$
— einen Oxiran-, Piperidino-, Morpholino-, Pyrrolidino- oder Furan-Ring,

$R_4$ einen $C_1$—$C_{12}$-Alkyl-Rest, der unsubstituiert oder gegebenenfalls substituiert ist durch $C_1$—$C_8$-Alkoxy, $C_2$—$C_8$-Alkoxycarbonyl, $C_3$—$C_8$-Cycloalkyl, gegebenenfalls auch durch einen unsubstituierten oder seinerseits durch Halogen, $C_1$—$C_4$-Alkyl, $C_1$—$C_4$-Alkoxy ein- oder mehrfach substituierten Phenyl-, Piperidino-, Morpholino-, Pyrrolidino- oder Furan-Ring,
— einen $C_3$—$C_{12}$-Alkenyl-Rest,
— einen gegebenenfalls durch $C_1$—$C_4$-Alkyl substituierten $C_3$—$C_{12}$-Cycloalkyl-Rest,
— einen Phenylrest, der unsubstituiert oder durch Halogen, $C_1$—$C_4$-Alkyl, $C_1$—$C_4$-Alkoxy, $C_1$—$C_4$-Alkylthio, —$NO_2$, —$CF_3$ ein- oder mehrfach substituiert ist,
— einen Piperidino-, Morpholino-, Pyrrolidino oder Furan-Ring,

$R_5$ und $R_6$ unabhängig voneinander je Wasserstoff,
— $C_1$—$C_8$-Alkyl, das durch Halogen, Hydroxyl oder Cyan substituiert und/oder dessen Kette durch Sauerstoff oder Carboxyloxy unterbrochen sein kann,
— $C_3$—$C_6$-Alkenyl, —Alkinyl oder —Cycloalkyl, Phenyl oder Benzyl, das durch Halogen, $C_1$—$C_4$-Alkyl, $C_1$—$C_4$-Alkoxy, —$NO_2$ oder —$CF_3$ substituiert sein kann,
und $R_6$ zusammen mit dem Stickstoffatom an das sie gebunden sind, auch einen Piperidino-, Morpholino- oder Pyrrolidino-Ring;
wobei die Bestimmung gilt, dass der Alkancarbonsäurerest

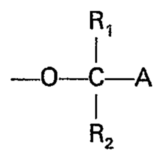

nicht in para-Stellung zu einem gegebenenfalls substituierten 2-Pyridyloxyrest stehen kann.

2. Die Verbindungen gemäss Anspruch 1 der Formel

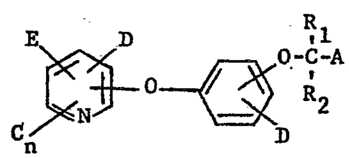

worin C und D je Halogen oder D Nitro, Cyano, $C_1$—$C_4$-Alkyl oder Thiocarbamid und E Wasserstoff oder Trifluormethyl bedeuten, während n, A, $R_1$ und $R_2$ die unter Formel I, Anspruch 1 gegebene Bedeutung haben.

3. Die Verbindungen gemäss Anspruch 1 der Formel

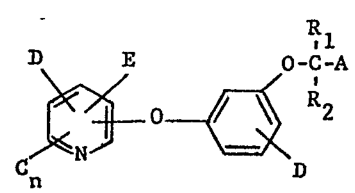

in denen C und D je Halogen oder D Nitro, Cyano, $C_1$—$C_4$-Alkyl oder Thiocarbamid und E Wasserstoff oder Trifluormethyl bedeuten, während n, A, $R_1$ und $R_2$ die unter Formel I, Anspruch 1 gegebene Bedeutung haben.

4. Die Verbindungen gemäss Anspruch 1 der Formel

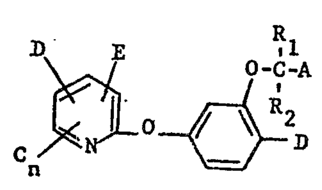

in denen C und D je Halogen oder D Nitro, Cyano, $C_1$—$C_4$-Alkyl oder Thiocarbamid und E Wasserstoff oder Trifluoromethyl bedeuten, während n, A, $R_1$ und $R_2$ die unter Formel I, Anspruch 1 gegebene Bedeutung haben.

5. Die Verbindungen gemäss Anspruch 1 der Formel

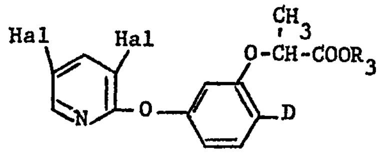

in denen "Hal" ein Halogenatom, D Halogen oder Nitro bedeuten und $R_3$ die unter Formel I im Anspruch 1 gegebene Bedeutung hat.

6. Die Verbindungen der Formel I, Anspruch 1, in denen

A eine Gruppe Cyano, COB oder

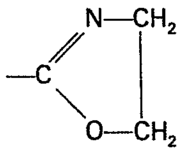

B einen Rest $—OR_3$, $—SR_4$, $—NR_5R_6$,
C Halogen,
D Wasserstoff, Halogen, $C_1$—$C_4$-Alkyl, Cyano oder Nitro, Thiocarbamid
E Wasserstoff, Halogen, Trifluormethyl, $C_1$—$C_4$-Alkyl, $C_1$—$C_4$-Alkoxycarbonyl, Nitro,
n 0, 1 oder 2,
$R_1$ Wasserstoff oder $C_1$—$C_4$-Alkyl,
$R_2$ Wasserstoff oder $C_1$—$C_4$-Alkyl,
$R_3$ Wasserstoff, das Kation eines Erdalkali- oder Alkalimetalls, eines quaternären Ammoniumsalzes, $C_1$—$C_6$-Alkyl, Phenyl gegebenenfalls substituiert durch Halogen, Cyan, Nitro oder $C_1$—$C_4$-Alkoxy,
$R_4$ $C_1$—$C_4$-Alkyl, -Alkenyl, -Alkinyl,
$R_5$ und $R_6$ einzeln je Wasserstoff oder $C_1$—$C_4$-Alkyl gegebenenfalls substituiert durch Halogen, Cyano oder Hydroxy,
$R_5$ und $R_6$ zusammen einen Piperidino-, Morpholino- oder Pyrrolidino-Ring darstellen.

7. α-[3-(3',5'-Dichlorpyridyl-2'-oxy)-6-chlorphenoxy]-propionsäure-methylester.

8. α-[3-(3',5'-Dichlorpyridyl-2'-oxy)-6-bromphenoxy]-propionsäure-methylester.

9. α-[3-(3',5'-Dichlorpyridyl-2'-oxy)-6-nitrophenoxy]-propionsäure-methylester.

10. α-[3-(3',5'-Dichlorpyridyl-2'-oxy)-6-chlorphenoxy]-propionsäure-isopropylester.

11. α-[3-(3',5'-Dichlorpyridyl-2'-oxy)-6-nitrophenoxy]-propionsäure-amid.

12. α-[3-(3',5'-Dichlorpyridyl-2'-oxy)-6-nitrophenoxy]-propionsäure-äthylamid.

13. Verfahren zur Herstellung der Pyridyloxy-phenoxy-alkancarbonsäurederivate der Formel I, Anspruch 1, dadurch gekennzeichnet, dass man in an sich bekannter Weise eine Verbindung der Formel II

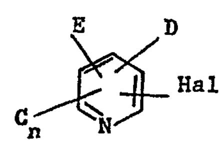

(II)

worin C, D, E und n die unter Formel I gegebene Bedeutung haben und Hal ein Halogenatom, vorzugsweise Chlor oder Brom bedeutet, mit einem Hydroxy - phenoxy - alkancarbonsäurederivat der Formel III

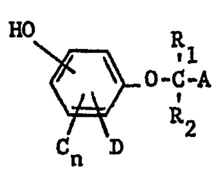

(III)

worin A, C, D, $R_1$ und $R_2$ die unter Formel I gegebene Bedeutung haben, in Gegenwart eines säurebindenden Mittels umsetzt.

14. Verfahren zur Herstellung der Pyridyloxy - phenoxy - alkancarbonsäurederivate der Formel I, Anspruch 1, dadurch gekennzeichnet, dass man in an sich bekannter Weise einen Pyridyloxy - hydroxyphenyläther der Formel IV

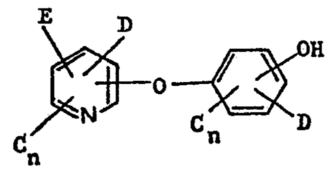

(IV)

worin C, D, E und n die unter Formel I gegebene Bedeutung haben, mit einem $\alpha$-Halogencarbonsäurederivat der Formel V

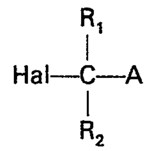

(V)

worin A, $R_1$ und $R_2$ die unter Formel I gegebene Bedeutung haben und Hal ein Halogenatom, vorzugsweise Chlor oder Brom bedeutet, in Gegenwart eines säurebindenden Mittels umsetzt.

15. Herbizides und das Pflanzenwachstum regulierendes Mittel, dadurch gekennzeichnet, dass es als Wirkstoff mindestens ein Pyridyloxy - phenoxy - alkancarbonsäurederivat der Formel I, Anspruch 1 enthält.

16. Die Verwendung der Pyridyloxy-phenoxy-alkancarbonsäurederivate der Formel I, Anspruch 1 oder sie enthaltender Mittel als Herbizide.

17. Die Verwendung der Pyridyloxy-phenoxy-alkancarbonsäurederivate der Formel I, Anspruch 1 oder sie enthaltender Mittel zur Beeinflussung des Pflanzenwachstums.

**Claims**

1. A pyridyloxy-phenoxyalkanecarboxylic acid derivative of the formula I

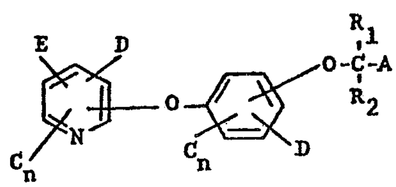

(I)

wherein

A is the cyano group, a radical —COB or a 2-oxazoline radical which is unsubstituted or substituted by one or more methyl groups,

B is a radical $—OR_3$, $—SR_4$ or $—NR_5R_6$,

C is halogen, $C_1—C_4$alkyl or nitro,

D is hydrogen, has the same meaning as C, or is $C_1—C_4$alkoxy, $C_1—C_4$alkylthio, cyano, $C_1—C_4$alkylsulfynyl, $C_1—C_4$alkylsulfonyl, thiocarbamide ($—CSNH_2$), mono or di-$C_1—C_4$alkylsulfamoyl,

E has the same meaning as C, or is $C_1—C_4$mono- or dialkylamine, $C_1—C_5$alkoxycarbonyl, $C_2—C_8$alkoxyalkyl, cyano, or trifluoromethyl,

n is 0, 1 or 2,

$R_1$ is hydrogen, $C_1—C_4$alkyl, $C_2—C_8$alkoxyalkyl, $C_1—C_4$haloalkyl,

$R_2$ is hydrogen, $C_1—C_4$alkyl, carbonyl, $C_1—C_5$alkoxycarbonyl,

$R_3$ is hydrogen or the cation of a base $\frac{1}{m}M^{m\oplus}$,

wherein M is an alkali metal cation or an alkaline earth metal cation or a Fe, Cu, Zn or

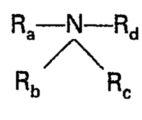

cation,

and m as integer 1, 2 or 3 corresponds to the valency of the cation, whilst $R_a$, $R_b$, $R_c$ und $R_d$, each independently of the other, are hydrogen, benzyl or a $C_1$—$C_4$alkyl radical which is unsubstituted or substituted by —OH, —$NH_2$ or $C_1$—$C_4$alkoxy; and

$R_3$ is further $C_1$—$C_8$alkoxy, $C_2$—$C_8$alkoxyalkoxy; $C_3$—$C_6$alkenyloxy, $C_1$—$C_8$alkylthio, $C_2$—$C_8$alkanoyl, $C_2$—$C_8$acyloxy, $C_2$—$C_8$alkoxycarbonyl, carbamoyl; bis($C_1$—$C_4$alkyl)amino, tris($C_1$—$C_4$alkyl)-ammonio, $C_3$—$C_8$cycloalkyl, $C_3$—$C_8$cycloalkenyl, and also by a phenyl, phenoxy or pyridyl, piperidyl, morpholyl, pyrolidinyl or furyl radical which is unsubstituted or is itself substituted by one or more halogen atoms, $C_1$—$C_4$alkyl or $C_1$—$C_4$alkoxy groups;
— a $C_3$—$C_{10}$alkenyl radical which is unsubstituted or substituted by 1 to 4 halogen atoms or monosubstituted by phenyl or methoxycarbonyl;
— a $C_3$—$C_8$alkynyl radical;
— a $C_3$—$C_{12}$cycloalkyl radical which is unsubstituted or substituted by halogen or $C_1$—$C_4$alkyl;
— a $C_3$—$C_8$cycloalkylene radical;
— a phenyl radical which is unsubstituted or substituted by one or more halogen atoms, $C_1$—$C_4$alkyl, $C_1$—$C_4$alkoxy, $C_1$—$C_4$alkylthio, $NO_2$, $CF_3$, COOH, CN, OH, $SO_3H$, $NH_2$ or —NH($C_1$—$C_4$alkyl) or —N($C_1$—$C_4$alkyl)$_2$ groups;
— an oxirane, piperidino, morpholino, pyrrolidino or furane ring;

$R_4$ is a $C_1$—$C_{12}$alkyl radical which is unsubstituted or substituted by $C_1$—$C_8$alkoxy, $C_2$—$C_8$alkoxy-carbonyl, $C_3$—$C_8$cycloalkyl, also by a phenyl, piperidino, morpholino, pyrrolidino or furane ring, which is unsubstituted or is itself substituted by one or more halogen atoms, $C_1$—$C_4$alkyl or $C_1$—$C_4$alkoxy groups;
— a $C_3$—$C_{12}$alkenyl radical;
— a $C_3$—$C_{12}$ cycloalkyl radical which is unsubstituted or substituted by $C_1$—$C_4$alkyl;
— a phenyl radical which is unsubstituted or substituted by one or more halogen atoms, $C_1$—$C_4$alkyl, $C_1$—$C_4$alkoxy, $C_1$—$C_4$alkylthio, $NO_2$ or $CF_3$ groups;
— a piperidino, morpholino, pyrrolidino or furane ring;

$R_5$ and $R_6$, each independently of the other, are $C_1$—$C_8$alkyl which can be substituted by halogen, hydroxyl or cyano and/or interrupted in the chain by oxygen or carboxyloxy,
— $C_3$—$C_6$alkenyl, alkynyl or cycloalkyl, phenyl or benzyl which can be substituted by halogen, $C_1$—$C_4$alkyl; $C_1$—$C_4$alkoxy, $NO_2$ or $CF_3$;
— or together with the nitrogen atom to which they are attached are also a piperidino, morpholino or pyrrolidino ring,

with the proviso that the alkanecarboxylic acid radical

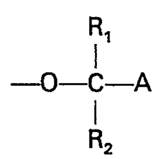

may not be in the para-position to an unsubstituted or substituted 2-pyridyloxy radical.

2. A compound according to claim 1 of the formula

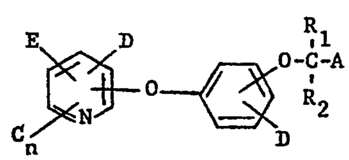

wherein each of C and D is halogen or D is nitro, cyano, $C_1$—$C_4$alkyl or thiocarbamide, and E is hydrogen or trifluoromethyl, whilst A, n, $R_1$ and $R_2$ are as defined for formula I in claim 1.

3. A compound according to claim 1 of the formula

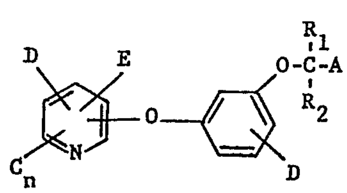

wherein each of C and D is halogen or D is nitro, cyano, $C_1$—$C_4$alkyl or thiocarbamide and E is hydrogen or trifluoromethyl, whilst A, n, $R_1$ and $R_2$ are as defined for formula I in claim 1.

4. A compound according to claim 1 of the formula

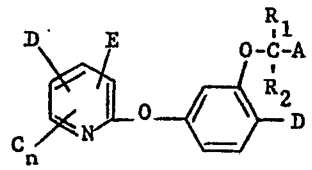

wherein each of C and D is halogen or D is nitro, cyano, $C_1$—$C_4$alkyl or thiocarbamide, and E is hydrogen or trifluoromethyl, whilst A, n, $R_1$ and $R_2$ are as defined for formula I in claim 1.

5. A compound according to claim 1 of the formula

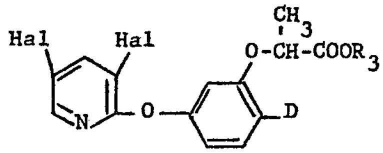

wherein "Hal" is halogen, D is halogen or nitro, whilst $R_3$ is as defined for formula I in claim 1.

6. A compound of the formula I according to claim 1, wherein

A is a cyano, —COB or

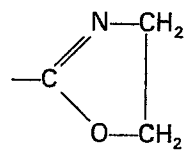

group,

B is a radical —$OR_3$, $SR_3$ or $NR_5R_6$,
C is halogen,
D is halogen, $C_1$—$C_4$alkyl, cyano, nitro or thiocarbamide,
E is hydrogen, halogen, trifluoromethyl, $C_1$—$C_4$alkyl, $C_1$—$C_4$alkoxycarbonyl or nitro,
n is 0, 1 or 2,
$R_1$ is hydrogen or $C_1$—$C_4$alkyl,
$R_2$ is hydrogen or $C_1$—$C_4$alkyl,
$R_3$ is hydrogen, the cation of an alkali metal or alkaline earth metal or of a quaternary ammonium salt, $C_1$—$C_6$alkyl, phenyl which is unsubstituted or substituted by halogen, cyano, nitro or $C_1$—$C_4$alkoxy,
$R_4$ is $C_1$—$C_4$alkyl, alkenyl or alkynyl,
$R_5$ and $R_6$, each independently of the other, is hydrogen or $C_1$—$C_4$alkyl which is unsubstituted or substituted by halogen, cyano, hydroxy or $C_1$—$C_4$alkyl,
$R_5$ and $R_6$ together are a piperidino, morpholino or pyrrolidino ring.

7. α-[3-(3′,5′-Dichloropyridyl-2′-oxy)-6-chlorophenoxy] propionic acid methyl ester.

8. α-[3-(3′,5′-Dichloropyridyl-2′-oxy)-6-bromophenoxy] propionic acid methyl ester.

9. α-[3-(3′,5′-Dichloropyridyl-2′-oxy)-6-nitrophenoxy] propionic acid methyl ester.

10. α-[3-(3′,5′-Dichloropyridyl-2′-oxy)-6-chlorophenoxy] propionic acid isopropyl ester.

11. α-[3-(3′,5′-Dichloropyridyl-2′-oxy)-6-nitrophenoxy] propionamide.

12. α-[3-(3′,5′-Dichloropyridyl-2′-oxy)-6-nitrophenoxy] propionic acid ethylamide.

13. A process for the manufacture of a novel pyridyloxyphenoxyalkanecarboxylic acid derivative of the formula I according to claim 1, characterised in that a compound of the formula II

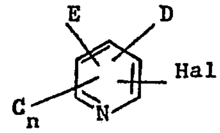

(II)

wherein C, D, E and n are as defined for formula I and Hal is a halogen atom, preferably chlorine or bromine, is reacted in a manner known per se with a hydroxyphenoxyalkanecarboxylic acid derivative of the formula III

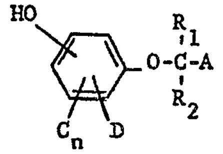

(III)

wherein A, C, D and $R_2$ are as defined for formula I, in the presence of an acid acceptor.

14. A process for the production of a pyridyloxyphenoxyalkanecarboxylic acid derivative of the formula I according to claim 1, characterised in that a pyridyloxyhydroxyphenyl ether of the formula IV

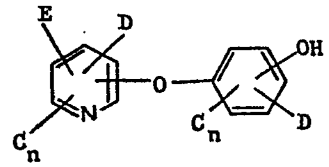

(IV)

wherein C, D, E and n are as defined for formula I, is reacted in a manner known per se with an $\alpha$-halocarboxylic acid derivative of the formula V

$$Hal-\overset{R_1}{\underset{R_2}{\overset{|}{\underset{|}{C}}}}-A \qquad (V)$$

wherein A, $R_1$ and $R_2$ are as defined for formula I and Hal is a halogen atom, preferably chlorine or bromine, in the presence of an acid acceptor.

15. A herbicidal and plant growth-regulating composition, characterised in that it contains, as active ingredient, at least one pyridyloxyphenoxyalkanecarboxylic acid derivative of the formula I according to claim 1.

16. Use of a pyridyloxyphenoxyalkanecarboxylic acid derivative of the formula I according to claim 1 or of a composition containing such a compound as herbicide.

17. Use of a pyridyloxyphenoxyalkanecarboxylic acid derivative of the formula I according to claim 1 or of a composition containing such a compound for influencing plant growth.

**Revendications**

1. Dérivés d'acides pyridyloxy-phénoxy-alcanoïques de formule I

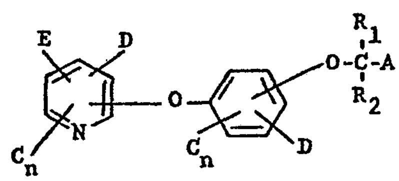

(I)

dans laquelle

A représente le groupe cyano, un reste —COB ou un reste de 2-oxazoline éventuellement mono- ou polysubstitué par des groupes méthyle,

B représente un reste $-OR_3$, $-SR_4$ ou $-NR_5R_6$, chacun des symboles C représente l'hydrogène, un halogène, un groupe alkyle en $C_1-C_4$ ou nitro, chacun des symboles D représente l'hydrogène, un substituant tel que ceux représentés par C, un groupe alcoxy en $C_1-C_4$, alkylthio en $C_1-C_4$, cyano, alkylsulfinyle en $C_1-C_4$, alkylsulfonyle en $C_1-C_4$, thiocarbamide ($-CSNH_2$), mono- ou di-(alkyle en $C_1-C_4$)-sulfamoyle,

E représente un substituant tel que ceux représentés par C, un groupe mono- ou di-(alkyle en $C_1-C_4$)-amine, (alcoxy en $C_1-C_5$)-carbonyle, alcoxyalkyle en $C_2-C_8$, cyano, trifluorométhyle,

n est égal à 0, 1 ou 2,

$R_1$ représente l'hydrogène, un groupe alkyle en $C_1-C_4$, alcoxyalkyle en $C_2-C_8$, halogénalkyle en $C_1-C_4$,

$R_2$ représente l'hydrogène, un groupe alkyle en $C_1-C_4$, carbonyle, (alcoxy en $C_1-C_5$)-carbonyle,

$R_3$ représente l'hydrogène ou le cation d'une base $\frac{1}{m} M^{m\oplus}$

M représentant un cation alcalin, alcalino-terreux, ou un cation Fe, Cu, Zn, 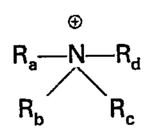

m est un nombre entier égal à 1, 2 ou 3 correspondant à la valence du cation, cependant que $R_a$, $R_b$, $R_c$ et $R_d$ représentent chacun, indépendamment les uns des autres, l'hydrogène, un groupe benzyle ou un reste alkyle portant éventuellement des substituants —OH, —$NH_2$ ou alcoxy en $C_1$—$C_4$,

en outre

$R_3$ représente un reste alkyle en $C_1$—$C_{18}$ non substitué ou éventuellement substitué par des halogènes, des groupes nitro, cyano, alcoxy en $C_1$—$C_8$, alcoxyalcoxy en $C_2$—$C_8$; alcényloxy en $C_3$—$C_6$, alkylthio en $C_1$—$C_8$, alcanoyle en $C_2$—$C_8$, acyloxy en $C_2$—$C_8$, alcoxycarbonyle en $C_2$—$C_8$, carbamoyle; bis-(alkyle en $C_1$—$C_4$)-amino, tris-(alkyle en $C_1$—$C_4$)-ammonio, cycloalkyle en $C_3$—$C_8$, cycloalcényle en $C_3$—$C_8$, le cas échéant également par un reste phényle, phénoxy, pyridyle, pipéridyle, morpholyle, pyrrolidinyle ou furyle non substitué ou lui-même mono- ou polysubstitué par des halogènes, des groupes alkyles en $C_1$—$C_4$, alcoxy en $C_1$—$C_4$,
— un reste alcényle en $C_3$—$C_{10}$ non substitué ou mono- à tétre-substitué par des halogènes ou un reste alcényle en $C_3$—$C_{10}$ substitué par un groupe phényle ou méthoxycarbonyle;
— un reste alcynyle en $C_3$—$C_8$;
— un reste cycloalkyle en $C_3$—$C_{12}$ éventuellement substitué par des halogènes ou des groupes alkyles en $C_1$—$C_4$;
— un reste cycloalkylène en $C_3$—$C_8$;
— un reste phényle non substitué ou mono- ou polysubstitué par des halogènes, des groupes alkyles en $C_1$—$C_4$, alcoxy en $C_1$—$C_4$, alkylthio en $C_1$—$C_4$, $NO_2$, $CF_3$, COOH, CN, OH, $SO_3H$, $NH_2$ ou NH-(alkyle en $C_1$—$C_4$) ou —N-(alkyle en $C_1$—$C_4)_2$;
— en outre $R_3$ représente un cycle oxirane, pipéridino, morpholino, pyrrolidino ou furanne,

$R_4$ représente un reste alkyle en $C_1$—$C_{12}$ non substitué ou éventuellement substitué par des groupes alcoxy en $C_1$—$C_8$, alcoxycarbonyle en $C_2$—$C_8$, cycloalkyle en $C_3$—$C_8$, éventuellement également par un cycle phényle, pipéridino, morpholino, pyrrolidino ou furanne non substitué ou lui-même mono- ou poly-substitué par des halogènes, des groupes alkyles en $C_1$—$C_4$, alcoxy en $C_1$—$C_4$;
— un reste alcényle en $C_3$—$C_{12}$;
— un reste cycloalkyle en $C_3$—$C_{12}$ éventuellement substitué par des groupes alkyles en $C_1$—$C_4$,
— un reste phényle non substitué ou mono- ou poly-substitué par des halogènes, des groupes alkyles en $C_1$—$C_4$, alcoxy en $C_1$—$C_4$, alkylthio en $C_1$—$C_4$, $NO_2$, $CF_3$;
— un cycle pipéridino, morpholino, pyrrolidino ou furanne;

$R_5$ et $R_6$ représentent chacun, indépendamment l'un de l'autre, l'hydrogène,
— un groupe alkyle en $C_1$—$C_8$ qui peut être substitué par des halogènes, des groupes hydroxy-ou cyano et/ou dont la chaîne peut être interrompue par l'oxygène ou un groupe carboxyloxy,
— un groupe alcényle en $C_3$—$C_6$, alcynyle ou cycloalkyle, phényle ou benzyle, qui peut être substitué par des halogènes, des groupes alkyles en $C_1$—$C_4$, alcoxy en $C_1$—$C_4$, $NO_2$ ou $CF_3$,

$R_5$ et $R_6$ peuvent également former avec l'atome d'azote auquel ils sont reliés un cycle pipéridino, morpholino ou pyrrolidino;

étant spécifié que le reste d'acide alcanoïque

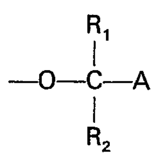

ne peut se trouver en position para d'un reste 2-pyridyloxy éventuellement substitué.

2. Les composés selon la revendication 1, de formule

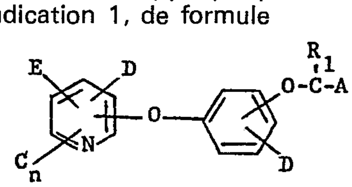

dans laquelle C et D représentent chacun un halogène ou bien D représente un groupe nitro, cyano, alkyle en $C_1$—$C_4$ ou thiocarbamide et E représente l'hydrogène ou un groupe trifluorométhyle, n, A, $R_1$ et $R_2$ ayant les significations indiquées en référence à la formule I dans la revendication 1.

3. Les composés selon la revendication 1, de formule

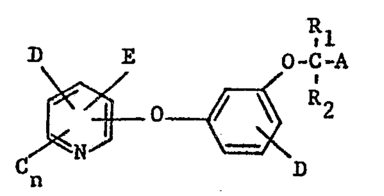

dans laquelle C et D représentent chacun un halogène ou D un groupe nitro, cyano, alkyle en $C_1$—$C_4$ ou thiocarbamide et E représente l'hydrogène ou un groupe trifluorométhyle, n, A, $R_1$ et $R_2$ ayant les significations indiquées en référence à la formule I dans la revendication 1.

4. Les composés selon la revendication 1, de formule

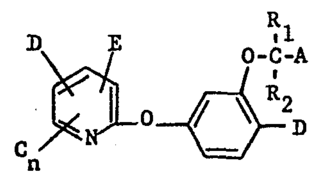

dans laquelle C et D représentent chacun un halogène ou D un groupe nitro, cyano, alkyle en $C_1$—$C_4$ ou thiocarbamide et E représente l'hydrogène ou un groupe trifluorométhyle, n, A, $R_1$ et $R_2$ ayant les significations indiquées en référence à la formule I dans la revendication 1.

5. Les composés selon la revendication 1, de formule

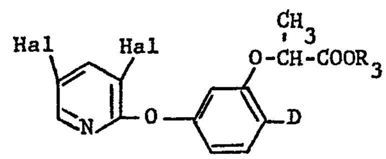

dans laquelle Hal représente un atome d'halogène, D représente un halogène ou un groupe nitro et $R_3$ a la signification indiquée en référence à la formule I dans la revendication 1.

6. Les composés de formule I, revendication 1, pour lesquels:

A représente un groupe cyano, COB ou

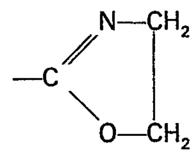

B représente un reste —$OR_3$, —$SR_4$, —$NR_5R_6$,
C représente un halogène,
D représente l'hydrogène, un halogène, un groupe alkyle en $C_1$—$C_4$, cyano ou nitro, thiocarbamide,
E représente l'hydrogène, un halogène, un groupe trifluorométhyle, alkyle en $C_1$—$C_4$, (alcoxy en $C_1$—$C_4$)-carbonyle ou nitro,
n est égal à 0, 1 ou 2,
$R_1$ représente l'hydrogène ou un groupe alkyle en $C_1$—$C_4$,
$R_2$ représente l'hydrogène ou un groupe alkyle en $C_1$—$C_4$,
$R_3$ représente l'hydrogène, le cation d'un métal alcalin ou alcalino-terreux, d'un sel d'ammonium quaternaire, un groupe alkyle en $C_1$—$C_6$, phényle éventuellement substitué par des halogènes, des groupes cyano, nitro ou alcoxy en $C_1$—$C_4$,
$R_4$ représente un groupe alkyle en $C_1$—$C_4$, alcényle, alcynyle,
$R_5$ et $R_6$ représentent chacun l'hydrogène ou un groupe alkyle en $C_1$—$C_4$ éventuellement substitué par des halogènes, des groupes cyano, hydroxy ou alcoxy en $C_1$—$C_4$,
$R_5$ et $R_6$ forment ensemble un cycle pipéridino, morpholino, pyrrolidino.

7. L'$\alpha$ - [3 - (3',5' - dichloropyridyl - 2' - oxy) - 6 - chlorophénoxy] - propionate de méthyle.

8. L'$\alpha$ - [3 - (3',5' - dichloropyridyl - 2' - oxy) - 6 - bromophénoxy] - propionate de méthyle.

9. L'$\alpha$ - [3 - (3',5' - dichloropyridyl - 2' - oxy) - 6 - nitrophénoxy] - propionate de méthyle.

10. L'$\alpha$ - [3 - (3',5' - dichloropyridyl - 2' - oxy) - 6 - chlorophénoxy] - propionate d'isopropyle.

11. L'$\alpha$ - [3 - (3',5' - dichloropyridyl - 2' - oxy) - 6 - nitrophénoxy] - propionamide.

12. L'$\alpha$ - [3 - (3',5' - dichloropyridyl - 2' - oxy) - 6 - nitrophénoxy] - propionéthylamide.

13. Procédé de préparation des nouveaux dérivés d'acides pyridyloxy - phénoxy - alcanoïques de formule I, revendication 1, caractérisé en ce que l'on fait réagir de manière connue en soi un composé de formule II

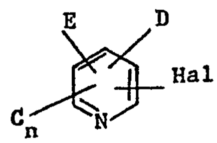

(II)

dans laquelle C, D, E et n ont les significations indiquées en référence à la formule I et Hal représente un atome d'halogène, de préférence de chlore ou de brome, avec un dérivé d'acide hydroxy - phénoxy - alcanoïque de formule III

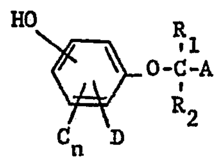

(III)

dans laquelle A, C, D, $R_1$ et $R_2$ ont les significations indiquées en référence à la formule I, en présence d'un agent fixant les acides.

14. Procédé de préparation de dérivés d'acides pyridyloxy - phénoxy - alcanoïques de formule I, revendication 1, caractérisé en ce que l'on fait réagir de manière connue en soi un éther pyridyloxy - hydroxy - phénylique de formule IV

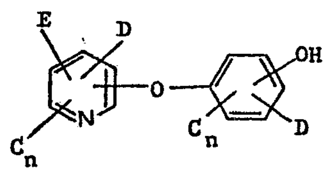

(IV)

dans laquelle C, D, E et n ont les significations indiquées en référence à la formule I, avec un dérivé d'acide $\alpha$-halogénocarboxylique de formule V

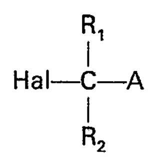

(V)

dans laquelle A, $R_1$ et $R_2$ ont les significations indiquées en référence à la formule I et Hal représente un atome d'halogène, de préférence de chlore ou de brome, en présence d'un agent fixant les acides.

15. Produit herbicide et régulateur de la croissance des végétaux, caractérisé en ce qu'il contient en tant que substance active au moins un dérivé d'acide pyridyloxy - phénoxy - alcanoïque de formule I, revendication 1.

16. L'utilisation des dérivés d'acides pyridyloxy - phénoxy - alcanoïques de formule I, revendication 1, ou de produits en contenant en tant qu'herbicides.

17. L'utilisation des dérivés d'acides pyridyloxy - phénoxy - alcanoïques de formule I, revendication 1, ou de produits en contenant pour agir sur la croissance des végétaux.